(19) Europäisches Patentamt
European Patent Office
Office européen des brevets

(11) EP 3 617 290 B1

(12) EUROPEAN PATENT SPECIFICATION

(45) Date of publication and mention of the grant of the patent:
**09.03.2022 Bulletin 2022/10**

(21) Application number: **19193630.1**

(22) Date of filing: **26.08.2019**

(51) International Patent Classification (IPC):
***C09K 11/06*** (2006.01) ***H01L 51/50*** (2006.01)
***C07D 403/14*** (2006.01) ***C07D 403/04*** (2006.01)
***H01L 51/00*** (2006.01)

(52) Cooperative Patent Classification (CPC):
**C09K 11/06; C07D 403/04; H01L 51/0067; H01L 51/0072;** C09K 2211/1007; C09K 2211/1029; C09K 2211/1059; H01L 51/5012; Y02E 10/549

(54) **ORGANIC MOLECULES FOR OPTOELECTRONIC DEVICES**

ORGANISCHE MOLEKÜLE FÜR OPTOELEKTRONISCHE VORRICHTUNGEN

MOLÉCULES ORGANIQUES POUR DISPOSITIFS OPTOÉLECTRONIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.08.2018 EP 18191507**

(43) Date of publication of application:
**04.03.2020 Bulletin 2020/10**

(73) Proprietor: **CYNORA GMBH**
**76646 Bruchsal (DE)**

(72) Inventors:
- **DIGENNARO, Dr. Angela**
  **69120 Heidelberg (DE)**
- **VELLAIYAPPAN, Arun Pandian**
  **76131 Karlsruhe (DE)**
- **BRANDT, Pascal**
  **76646 Bruchsal (DE)**

(74) Representative: **Hoppe, Georg Johannes**
**Darani Anwaltskanzlei**
**Beuckestrasse 20**
**14163 Berlin (DE)**

(56) References cited:
**EP-A1- 3 015 465**
**EP-A1- 3 315 581**
**DE-B3-102016 120 373**
**TW-A- 201 825 459**
**TW-B- I 648 262**
**US-A1- 2014 145 149**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 617 290 B1

## Description

The invention relates to light-emitting organic molecules and their use in organic light-emitting diodes (OLEDs) and in other optoelectronic devices.

## Description

The object of the present invention is to provide molecules which are suitable for use in optoelectronic devices.
This object is achieved by the invention which provides a new class of organic molecules.
The organic molecules of the invention are preferably purely organic molecules, i.e. they do not contain any metal ions, which is in contrast to metal complexes known for use in optoelectronic devices. Therefore, according to the present invention, it is preferred that the organic molecules are free of metal atoms or metal ions. EP3315581 A1 discloses molecules similar to the present invention.
According to the present invention, the organic molecules exhibit emission maxima in the blue, sky-blue or green spectral range. The organic molecules exhibit in particular emission maxima between 420 nm and 520 nm, preferably between 440 nm and 495 nm, more preferably between 450 nm and 470 nm. The photoluminescence quantum yields of the organic molecules according to the invention are, in particular, 20% or more. The molecules according to the invention exhibit in particular thermally activated delayed fluorescence (TADF). The use of the molecules according to the invention in an optoelectronic device, for example an organic light-emitting diode (OLED), leads to higher efficiencies of the device. Corresponding OLEDs have a higher stability than OLEDs with known emitter materials and comparable color.
The organic molecules of the invention comprise or consist of a structure of Formula I,

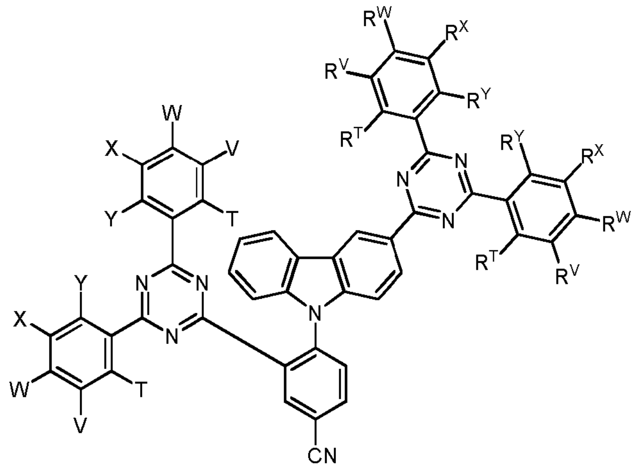

Formula I

wherein

T, V, W, X, Y is independently from each other selected from the group consisting of H and tert-butyl; and

$R^T$, $R^V$, $R^W$, $R^X$, $R^Y$ is independently from each other selected from the group consisting of H and tert-butyl;

wherein at least one kind of substituents selected from the group consisting of T, V, W, X, Y, $R^T$, $R^V$, $R^W$, $R^X$, $R^Y$ is tert-butyl at each occurrence. Since Formula I shows two substituents of each kind, either none of the two or both substituents of that one kind are tert-butyl.
In one embodiment of the invention, exactly one kind of substituent, exactly two kinds of substituents, exactly three kinds of substituents, or exactly four kinds of substituents is/are selected from the group consisting of T, V, W, X, Y, $R^T$, $R^V$, $R^W$, $R^X$, $R^Y$ is tert-butyl. Accordingly, the organic molecule has an even number of tert-butyl groups.
In one embodiment of the invention, exactly one kind of substituents selected from the group consisting of T, V, W, X, Y, $R^T$, $R^V$, $R^W$, $R^X$, $R^Y$ is tert-butyl.
In one embodiment of the invention, exactly two kinds of substituents selected from the group consisting of T, V, W, X, Y, $R^T$, $R^V$, $R^W$, $R^X$, $R^Y$ are tert-butyl.
In some embodiments of the invention, exactly one kind of substituents selected from the group consisting of

T, V, W, X, Y is tert-butyl and
exactly one kind of substituents selected from the group consisting of $R^T$, $R^V$, $R^W$, $R^X$, $R^Y$ is tert-butyl.

In one embodiment of the invention, exactly two kinds of substituents selected from the group consisting of T, V, W, X, Y are tert-butyl at each occurrence, and
exactly two kinds of substituents selected from the group consisting of $R^T$, $R^V$, $R^W$, $R^X$, $R^Y$ $_{are}$ tert-butyl.

In further embodiments of the invention, the organic molecules of the invention comprise or consist of a structure of Formula Ia:

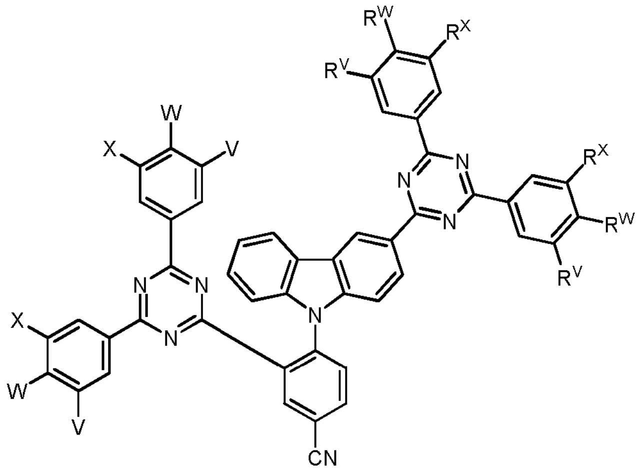

Formula Ia

wherein

V, W, X is independently from each other selected from the group consisting of H and tert-butyl; and
$R^V$, $R^W$, $R^X$ is independently from each other selected from the group consisting of H and tert-butyl;

wherein at least one kind of substituents selected from the group consisting of V, W, X, $R^V$, $R^W$, $R^X$ is tert-butyl.

In further embodiments of the invention, the organic molecules of the invention comprise or consist of a structure of Formula Iaa:

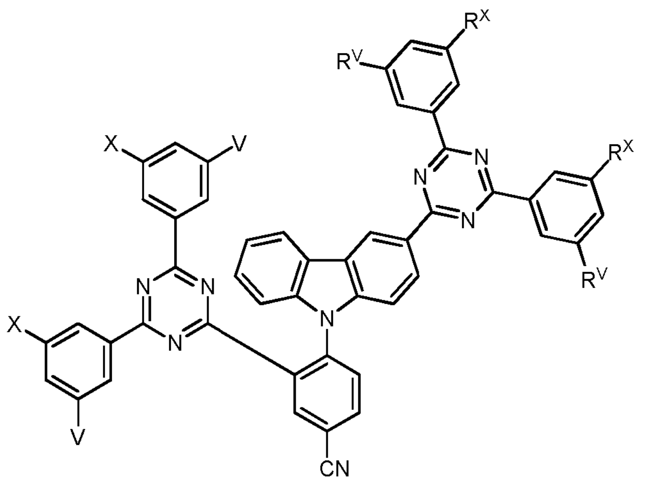

Formula Iaa

wherein
V, X is independently from each other selected from the group consisting of H and tert-butyl; $R^V$, $R^X$ is independently from each other selected from the group consisting of H and tert-butyl; wherein at least one kind of substituents selected

from the group consisting of V, X, $R^V$, $R^X$ is tert-butyl.

In further embodiments of the invention, the organic molecules of the invention comprise or consist of a structure of Formula Iaaa:

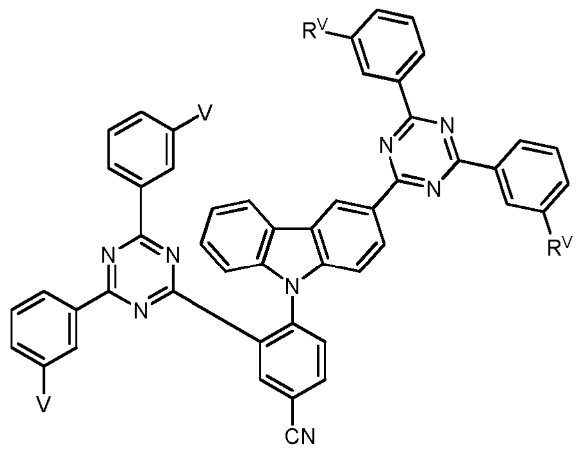

Formula Iaaa

wherein

V is selected from the group consisting of H and tert-butyl;
$R^V$ is selected from the group consisting of H and tert-butyl;
wherein at least one kind of substituents selected from the group consisting of V, $R^V$ is tert-butyl.

In some embodiment, the organic molecules of the invention comprise or consist of a structure selected from the group consisting of Formula II, Formula III, Formula IV or Formula V.

In further embodiments of the invention, the organic molecules comprise or consist of a structure of Formula II:

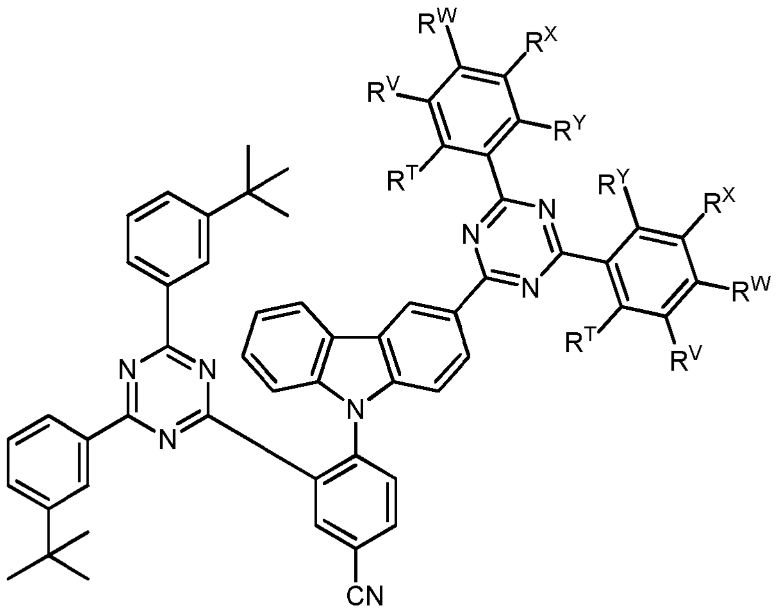

Formula II

wherein any one of the aforementioned definitions apply.

In further embodiments, the organic molecules of the invention comprise or consist of a structure selected from the group consisting of Formula IIa, Formula IIb, Formula IIc, Formula Iid, Formula Iie, Formula IIf and Formula IIg:

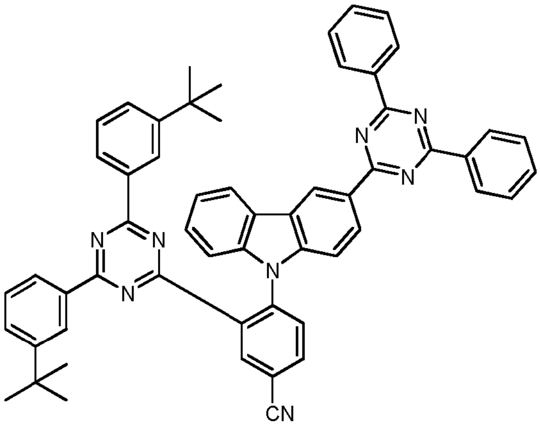

Formula IIa

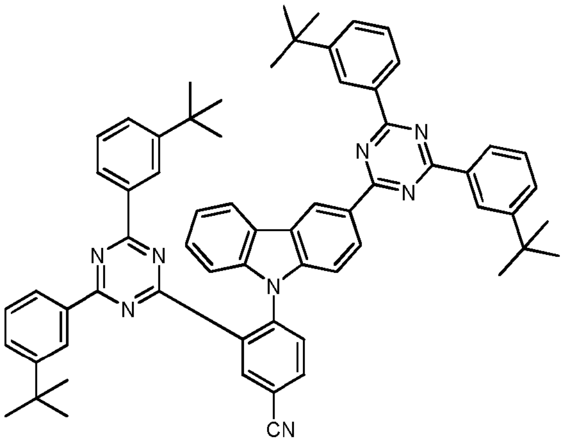

Formula IIb

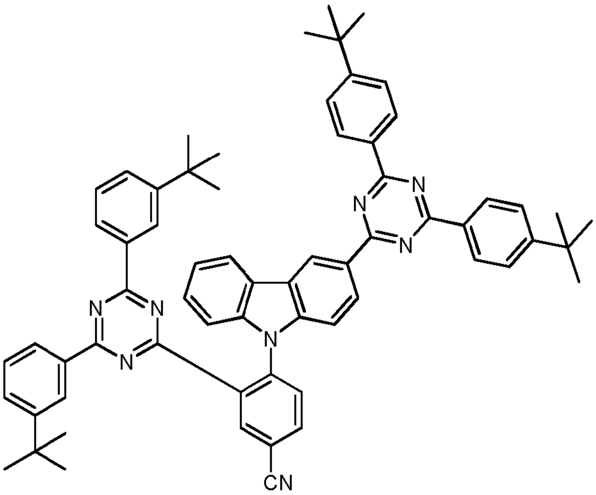

Formula IIc

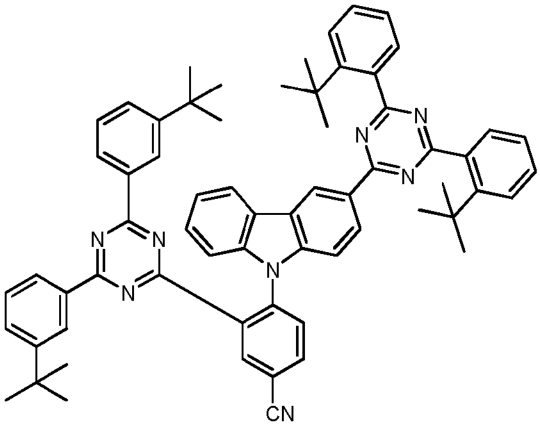

Formula IId

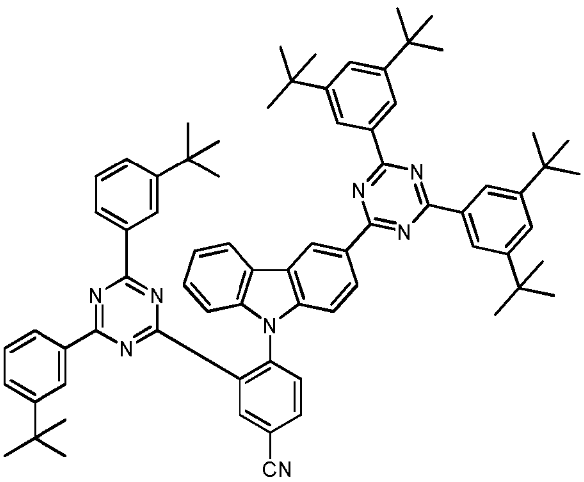

Formula IIe

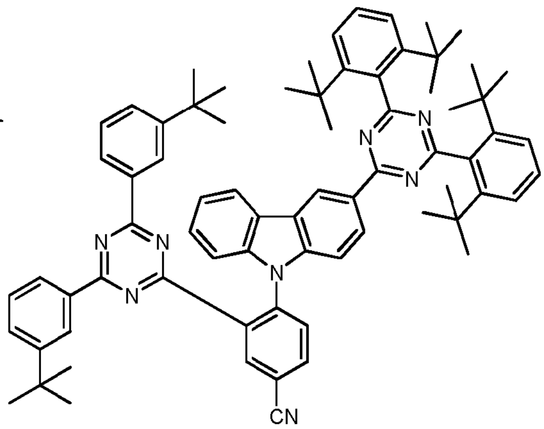

Formula IIf

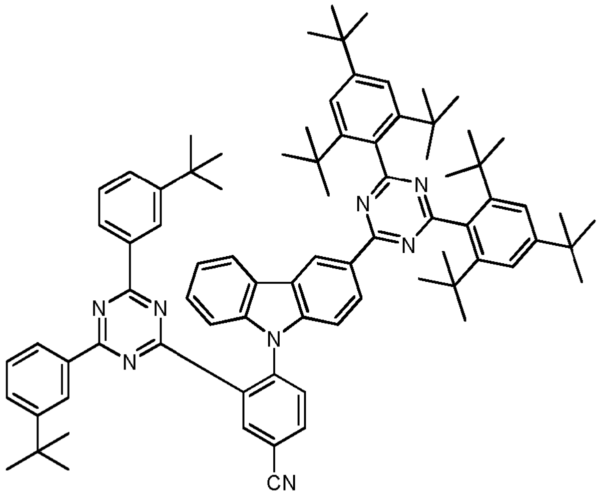

Formula IIg

In further embodiments, the organic molecules of the invention comprise or consist of a structure of Formula III:

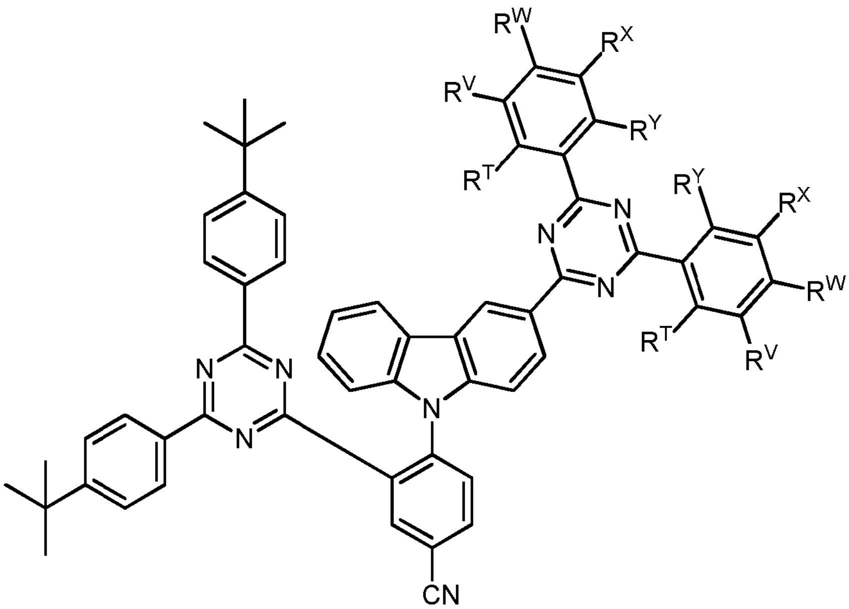

Formula III

wherein any one of the aforementioned definitions apply.

In a further embodiment of the invention, the organic molecules comprise or consist of a structure selected from the group consisting of Formula IIIa, Formula IIIb, Formula IIIc, Formula IIId, Formula IIIe, Formula IIIf and Formula IIIg:

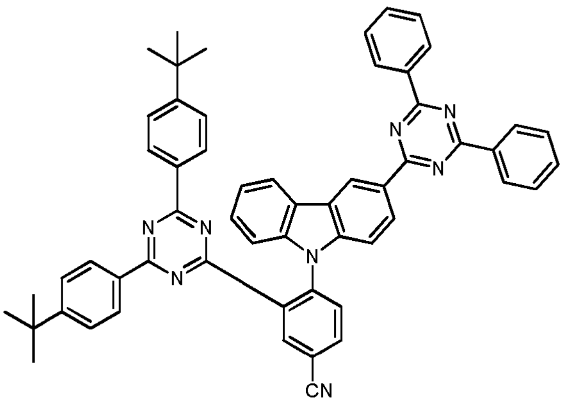

Formula IIIa

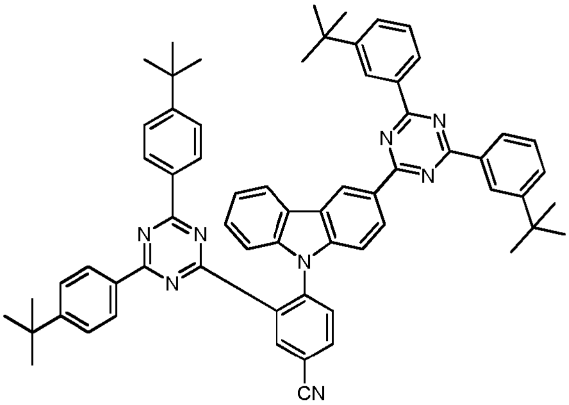

Formula IIIb

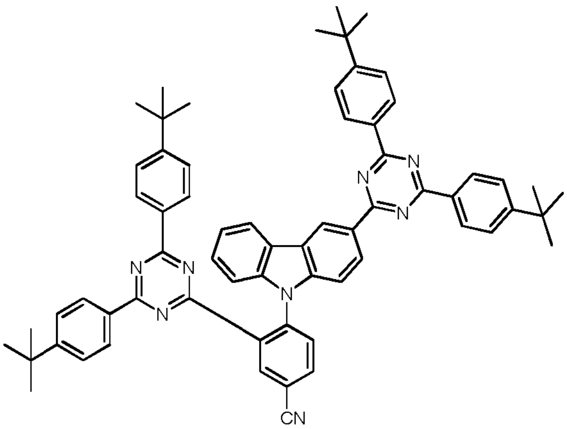

Formula IIIc

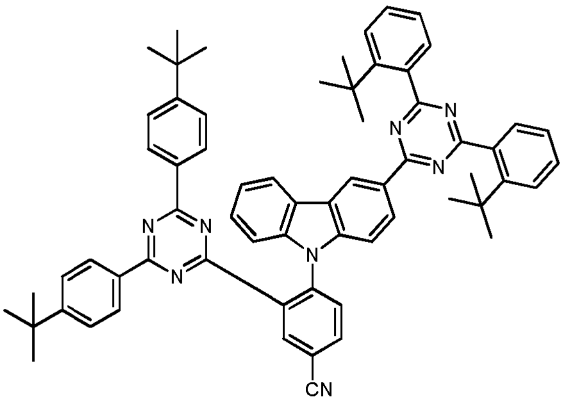

Formula IIId

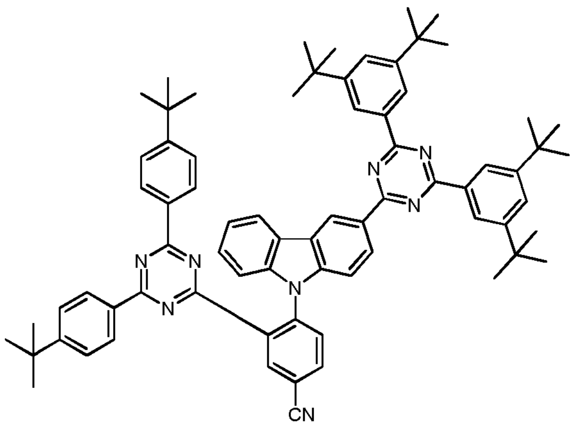

Formula IIIe

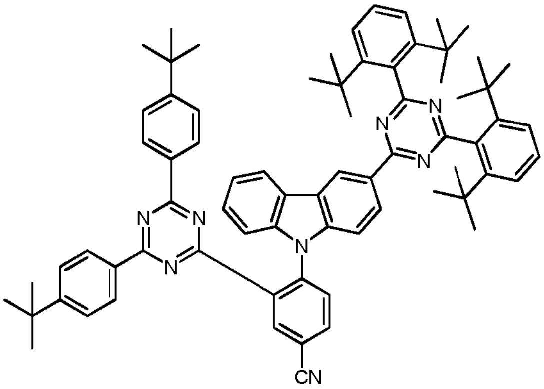

Formula IIIf

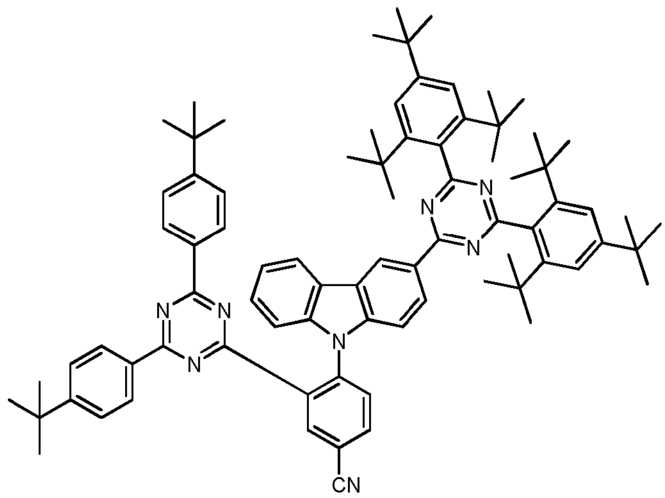

Formula IIIg

In further embodiments, the organic molecules of the invention comprise or consist of a structure of Formula IV:

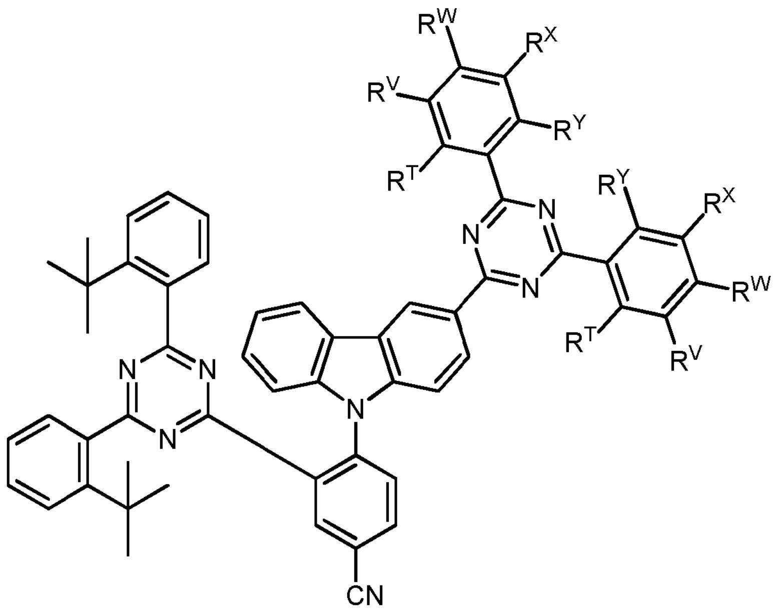

Formula IV

wherein any one of the aforementioned definitions apply.

In further embodiments, the organic molecules of the invention comprise or consist of a structure selected from the group consisting of Formula IVa, Formula IVb, Formula IVc, Formula IVd, Formula IVe, Formula IVf and Formula IVg:

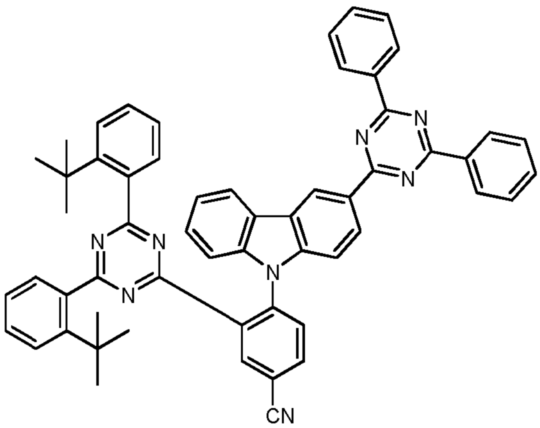

Formula IVa

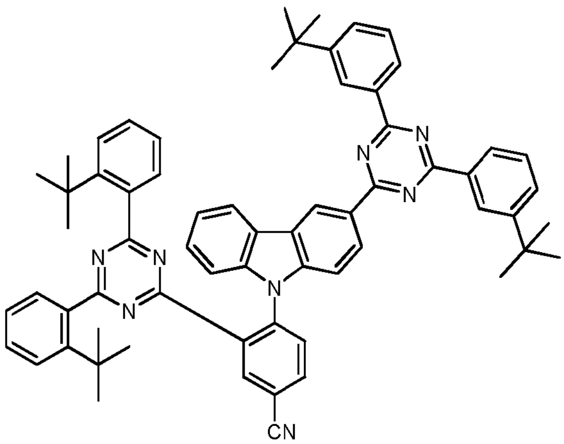

Formula IVb

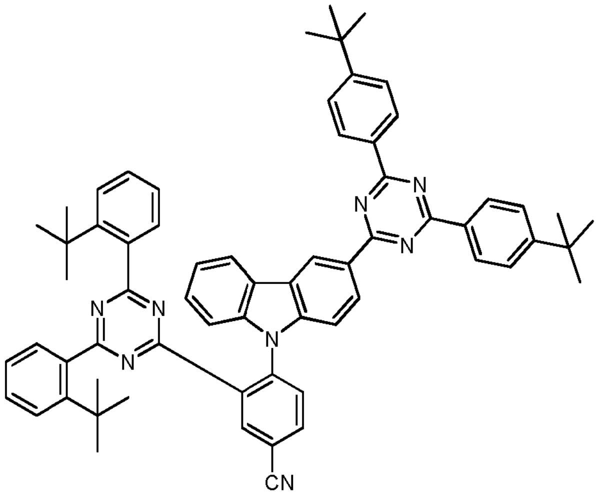

Formula IVc

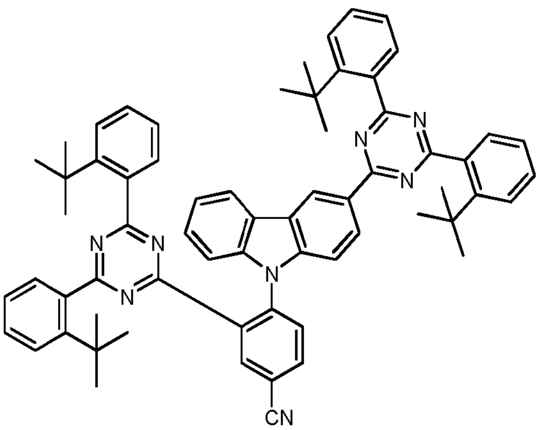

Formula IVd

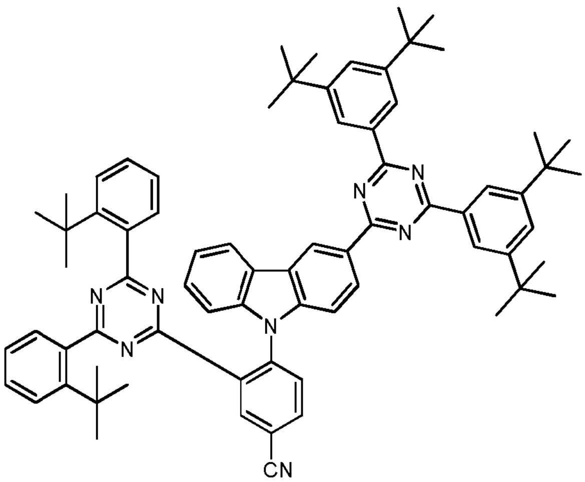

Formula IVe

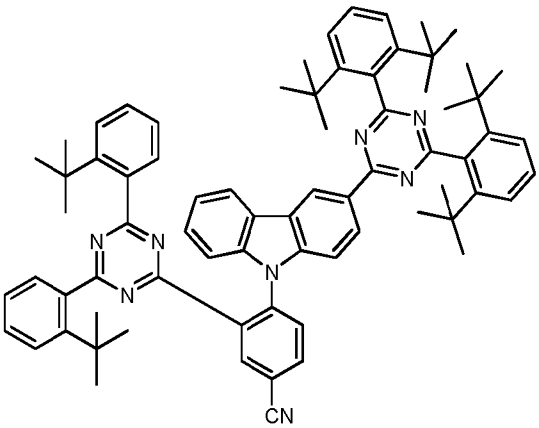

Formula IVf

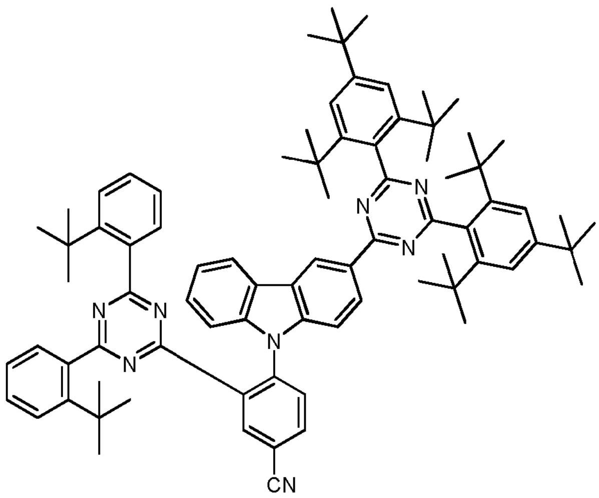

Formula IVg

In further embodiments of the invention, the organic molecules comprise or consist of a structure of Formula V:

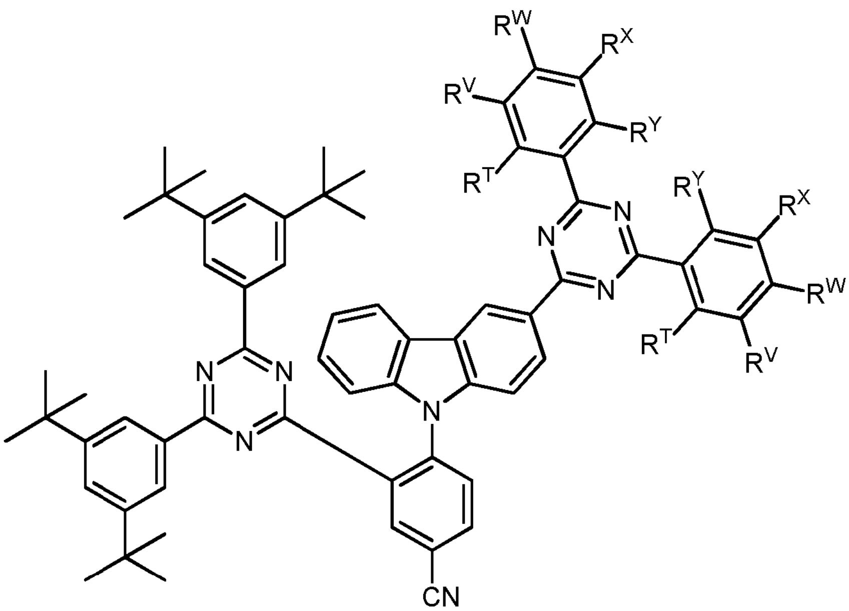

Formula V

wherein any one of the aforementioned definitions apply.

In further embodiments of the invention, the organic molecules of the invention comprise or consist of a structure selected from the group consisting of Formula Va, Formula Vb, Formula Vc, Formula Vd, Formula Ve, Formula Vf and Formula Vg:

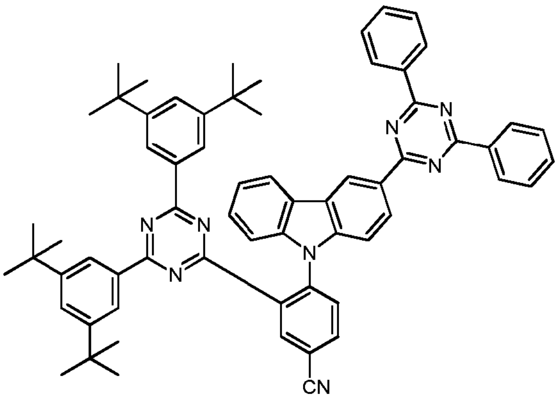

Formula Va

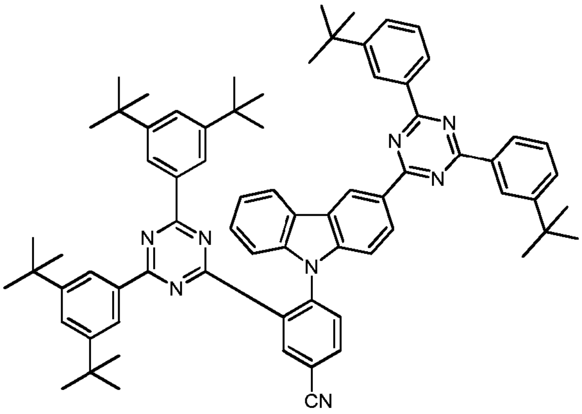

Formula Vb

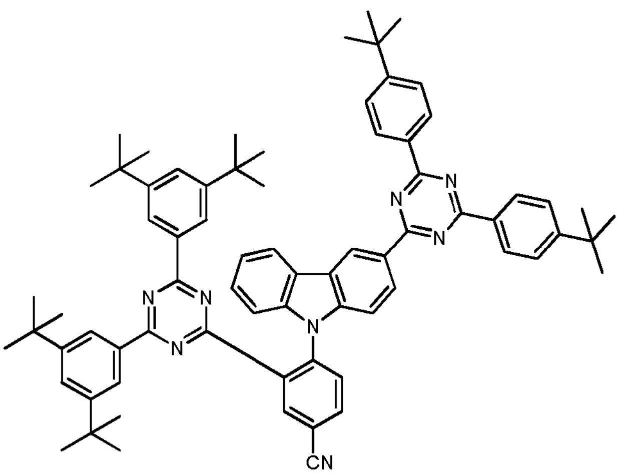

Formula Vc

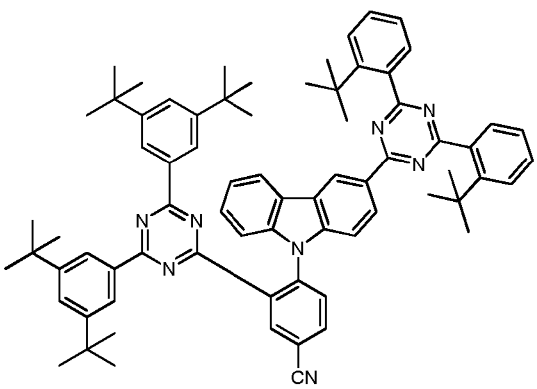

Formula Vd

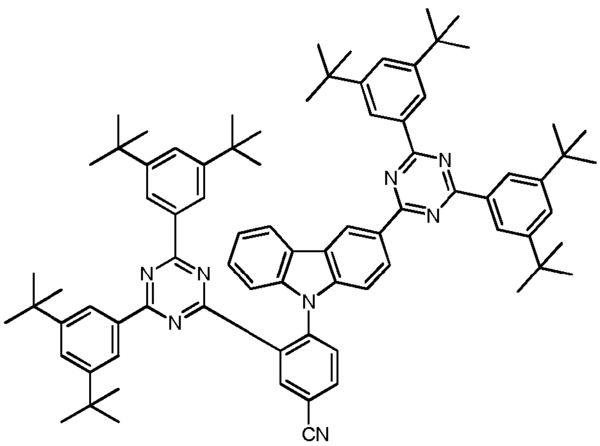

Formula Ve

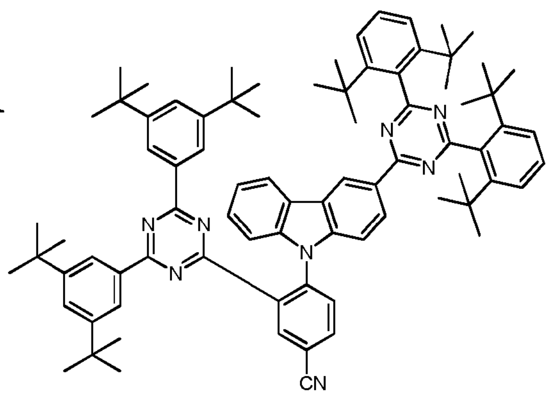

Formula Vf

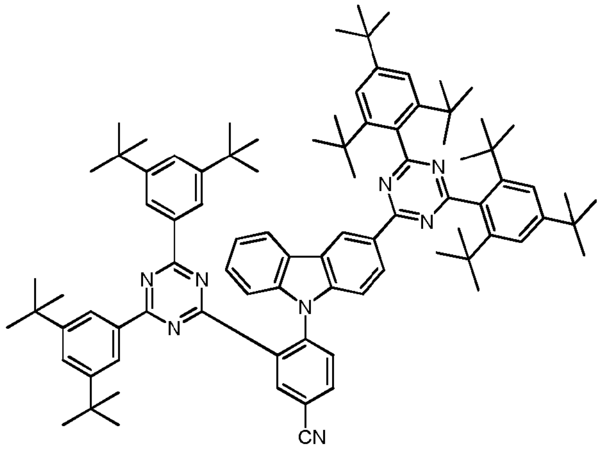

Formula Vg

In further embodiments of the invention, the organic molecules comprise or consist of a structure of Formula VI:

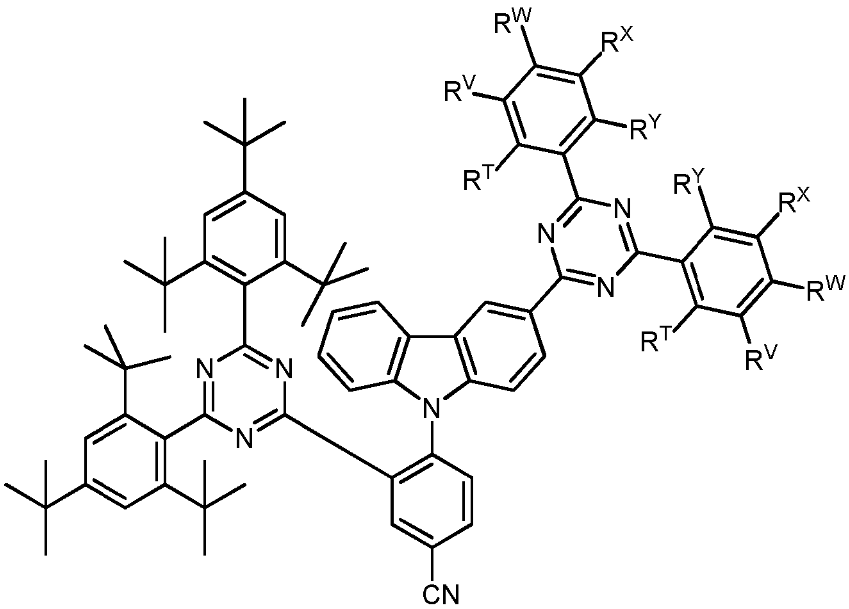

Formula VI

wherein any one of the aforementioned definitions apply.

In a further embodiment of the invention, the organic molecules of the invention comprise or consist of a structure selected from the group consisting of Formula Via, Formula VIb, Formula VIc, Formula VId, Formula Vie, Formula VIf and Formula VIg:

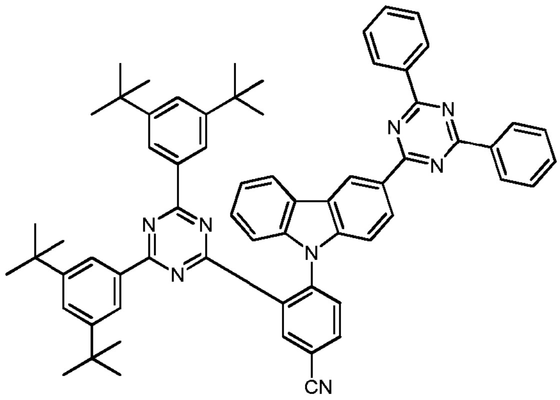

Formula VIa

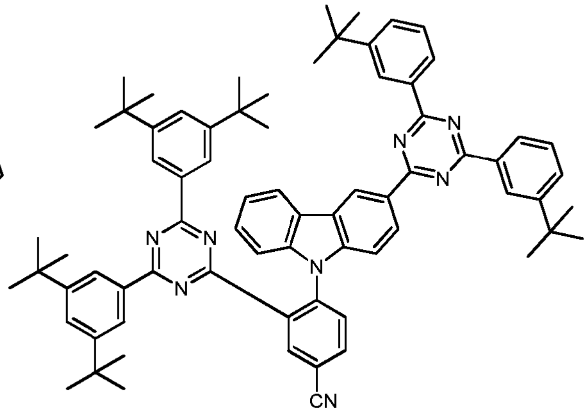

Formula VIb

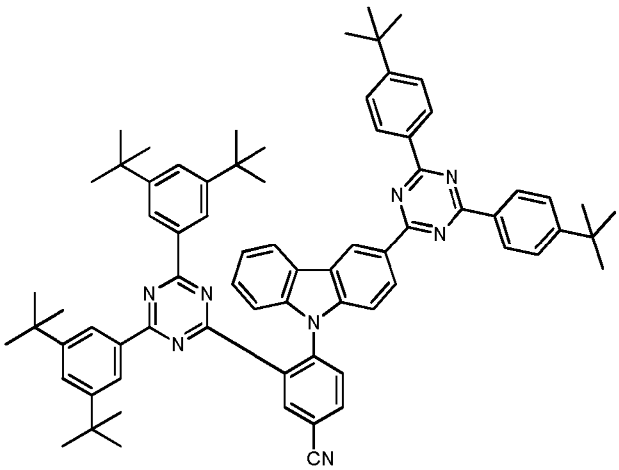

Formula VIc

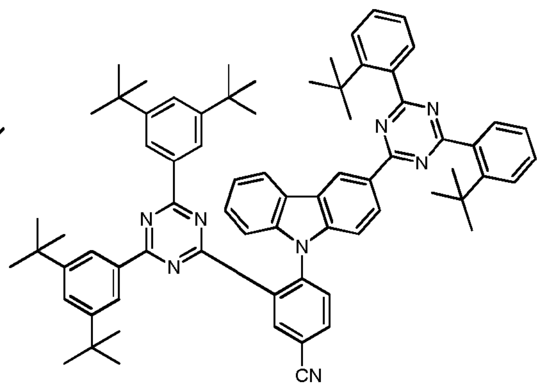

Formula VId

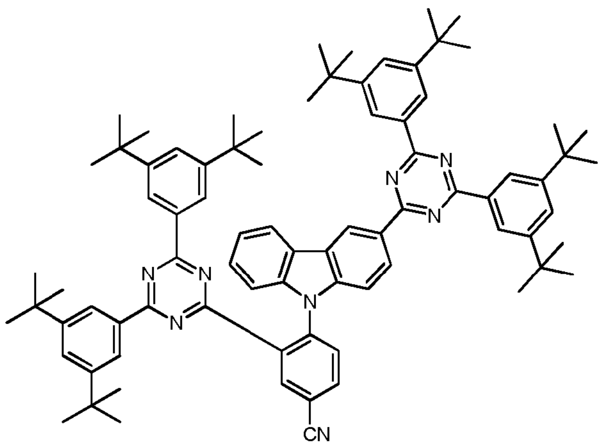

Formula VIe

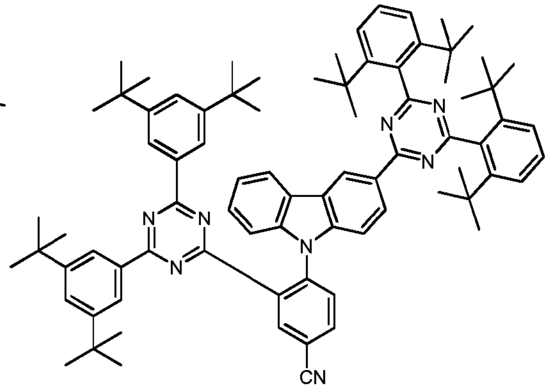

Formula VIf

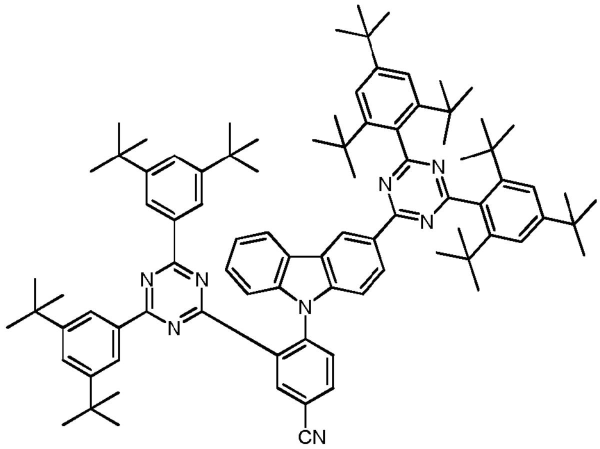

Formula VIg

In a further embodiment of the invention, the organic molecules of the invention comprise or consist of a structure of Formula VII:

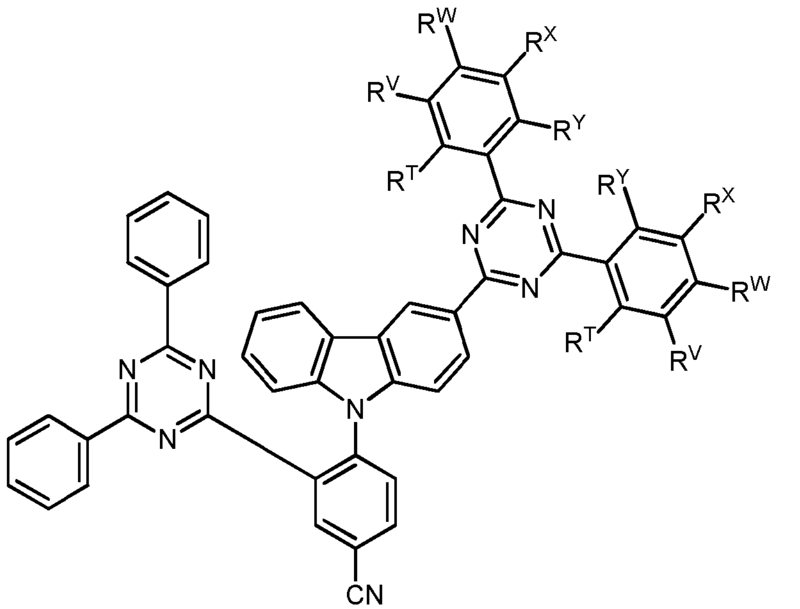

Formula VII

wherein any one of the aforementioned definitions apply.

In further embodiments of the invention, the organic molecules of the invention comprise or consist of a structure selected from the group consisting of Formula Vila, Formula Vllb, Formula VIIc, Formula Vlld, Formula Vile and Formula Vllf:

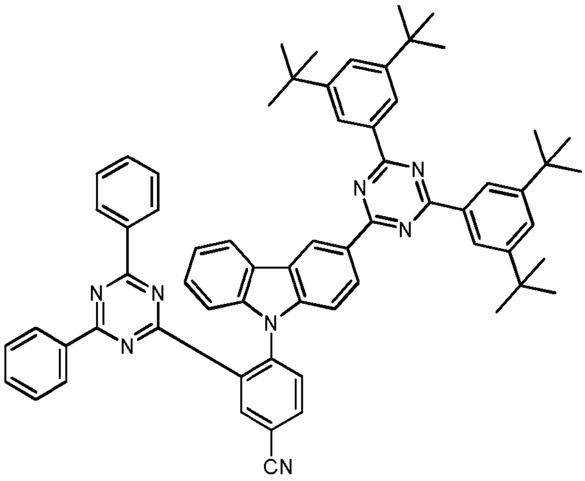

Formula VIIa

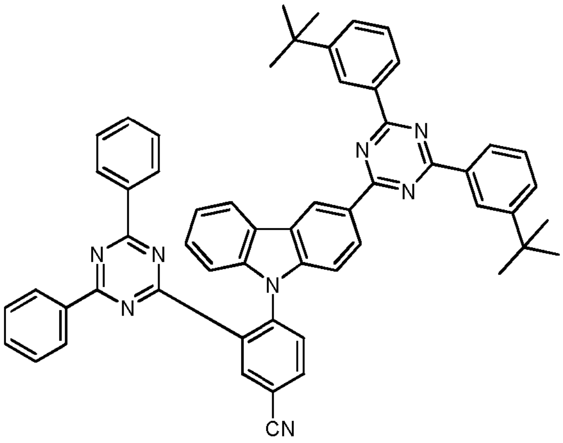

Formula VIIb

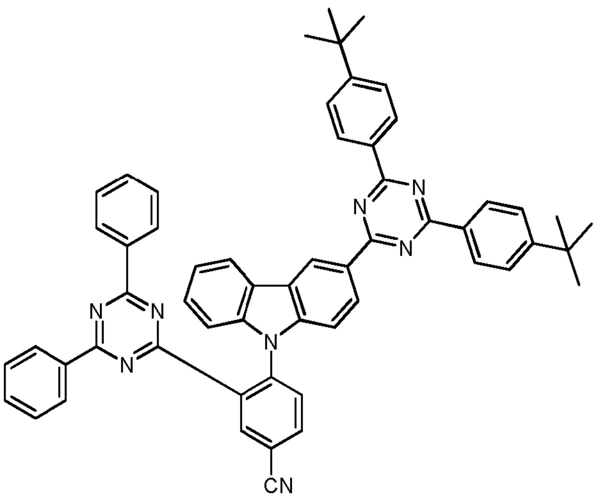

Formula VIIc

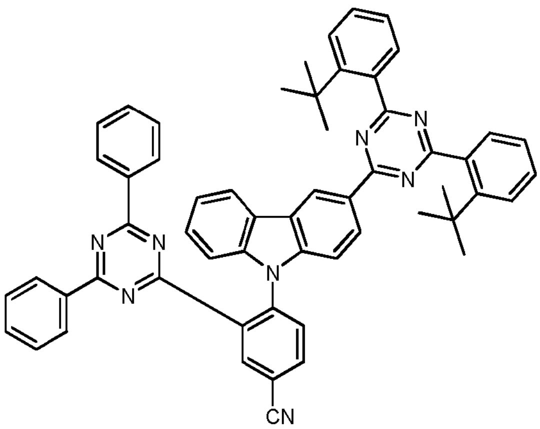

Formula VIId

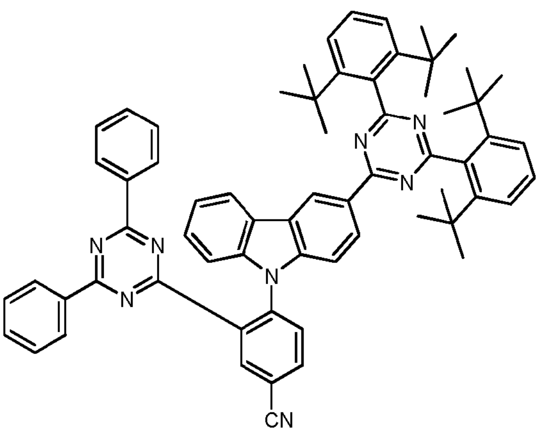

Formula VIIe

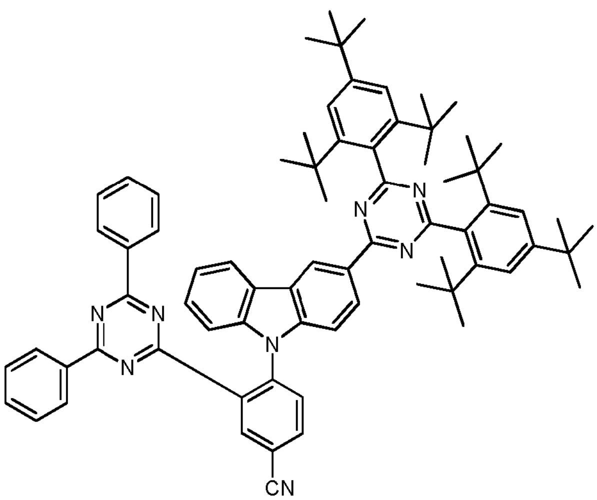

Formula VIIf

As used throughout, the terms "aryl" and "aromatic" may be understood in the broadest sense as any mono-, bi- or polycyclic aromatic moieties. Accordingly, an aryl group contains 6 to 60 aromatic ring atoms, and a heteroaryl

group contains 5 to 60 aromatic ring atoms, of which at least one is a heteroatom. Notwithstanding, throughout the application the number of aromatic ring atoms may be given as subscripted number in the definition of certain substituents. In particular, the heteroaromatic ring includes one to three heteroatoms. Again, the terms "heteroaryl" and "heteroaromatic" may be understood in the broadest sense as any mono-, bi- or polycyclic hetero-aromatic moieties that include at least one heteroatom. The heteroatoms may at each occurrence be the same or different and be individually selected from the group consisting of N, O and S. Accordingly, the term "arylene" refers to a divalent substituent that bears two binding sites to other molecular structures and thereby serving as a linker structure. In case, a group in the exemplary embodiments is defined differently from the definitions given here, for example, the number of aromatic ring atoms or number of heteroatoms differs from the given definition, the definition in the exemplary embodiments is to be applied. According to the invention, a condensed (annulated) aromatic or heteroaromatic polycycle is built of two or more single aromatic or heteroaromatic cycles, which formed the polycycle via a condensation reaction.

In particular, as used throughout, the term aryl group or heteroaryl group comprises groups which can be bound via any position of the aromatic or heteroaromatic group, derived from benzene, naphthaline, anthracene, phenanthrene, pyrene, dihydropyrene, chrysene, perylene, fluoranthene, benzanthracene, benzphenanthrene, tetracene, pentacene, benzpyrene, furan, benzofuran, isobenzofuran, dibenzofuran, thiophene, benzothiophene, isobenzothiophene, dibenzothiophene; pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthoimidazole, phenanthroimidazole, pyridoimidazole, pyrazinoimidazole, quinoxalinoimidazole, oxazole, benzoxazole, napthooxazole, anthroxazol, phenanthroxazol, isoxazole, 1,2-thiazole, 1,3-thiazole, benzothiazole, pyridazine, benzopyridazine, pyrimidine, benzopyrimidine, 1,3,5-triazine, quinoxaline, pyrazine, phenazine, naphthyridine, carboline, benzocarboline, phenanthroline, 1,2,3-triazole, 1,2,4-triazole, benzotriazole, 1,2,3-oxadiazole, 1,2,4-oxadiazole, 1,2,5-oxadiazole, 1,2,3,4-tetrazine, purine, pteridine, indolizine and benzothiadiazole or combinations of the abovementioned groups.

As used throughout, the term cyclic group may be understood in the broadest sense as any mono-, bi- or polycyclic moieties.

As used throughout the present description, the term biphenyl as a substituent may be understood in the broadest sense as ortho-biphenyl, meta-biphenyl, or para-biphenyl, wherein ortho, meta and para is defined in regard to the binding site to another chemical moiety.

As used throughout the present description, the term alkyl group may be understood in the broadest sense as any linear, branched, or cyclic alkyl substituent. In particular, the term alkyl comprises the substituents methyl (Me), ethyl (Et), n-propyl ($^{n}$Pr), i-propyl ($^{i}$Pr), cyclopropyl, n-butyl ($^{n}$Bu), i-butyl ($^{i}$Bu), s-butyl ($^{s}$Bu), t-butyl ($^{t}$Bu), cyclobutyl, 2-methylbutyl, n-pentyl, s-pentyl, t-pentyl, 2-pentyl, neo-pentyl, cyclopentyl, n-hexyl, s-hexyl, t-hexyl, 2-hexyl, 3-hexyl, neohexyl, cyclohexyl, 1-methylcyclopentyl, 2-methylpentyl, n-heptyl, 2-heptyl, 3-heptyl, 4-heptyl, cycloheptyl, 1-methylcyclohexyl, n-octyl, 2-ethylhexyl, cyclooctyl, 1-bicyclo[2,2,2]octyl, 2-bicyclo[2,2,2]-octyl, 2-(2,6-dimethyl)octyl, 3-(3,7-dimethyl)octyl, adamantyl, 2,2,2-trifluorethyl, 1,1-dimethyl-n-hex-1-yl, 1,1-dimethyl-n-hept-1-yl, 1,1-dimethyl-n-oct-1-yl, 1,1-dimethyl-n-dec-1-yl, 1,1-dimethyl-n-dodec-1-yl, 1,1-dimethyl-n-tetradec-1-yl, 1,1-dimethyl-n-hexadec-1-yl, 1,1-dimethyl-n-octadec-1-yl, 1,1-diethyl-n-hex-1-yl, 1,1-diethyl-n-hept-1-yl, 1,1-diethyl-n-oct-1-yl, 1,1-diethyl-n-dec-1-yl, 1,1-diethyl-n-dodec-1-yl, 1,1-diethyl-n-tetradec-1-yl, 1,1-diethyln-n-hexadec-1-yl, 1,1-diethyl-n-octadec-1-yl, 1-(n-propyl)-cyclohex-1-yl, 1-(n-butyl)-cyclohex-1-yl, 1-(n-hexyl)-cyclohex-1-yl, 1-(n-octyl)-cyclohex-1-yl and 1-(n-decyl)-cyclohex-1-yl.

As used throughout, the term alkenyl comprises linear, branched, and cyclic alkenyl substituents. The term alkenyl group exemplarily comprises the substituents ethenyl, propenyl, butenyl, pentenyl, cyclopentenyl, hexenyl, cyclohexenyl, heptenyl, cycloheptenyl, octenyl, cyclooctenyl or cyclooctadienyl.

As used throughout, the term alkynyl comprises linear, branched, and cyclic alkynyl substituents. The term alkynyl group exemplarily comprises ethynyl, propynyl, butynyl, pentynyl, hexynyl, heptynyl or octynyl.

As used throughout the present application, the term alkoxy comprises linear, branched, and cyclic alkoxy substituents. The term alkoxy group exemplarily comprises methoxy, ethoxy, n-propoxy, i-propoxy, n-butoxy, i-butoxy, s-butoxy, t-butoxy and 2-methylbutoxy.

As used throughout the present application, the term thioalkoxy comprises linear, branched, and cyclic thioalkoxy substituents, in which the O of the exemplarily alkoxy groups is replaced by S.

As used throughout, the terms "halogen" and "halo" may be understood in the broadest sense as being preferably fluorine, chlorine, bromine or iodine.

Whenever hydrogen (H) is mentioned herein, it could also be replaced by deuterium at each occurrence.

It is understood that when a molecular fragment is described as being a substituent or otherwise attached to another moiety, its name may be written as if it were a fragment (e.g. naphtyl, dibenzofuryl) or as if it were the whole molecule (e.g. naphthalene, dibenzofuran). As used herein, these different ways of designating a substituent or attached fragment are considered to be equivalent.

The organic molecules according to the invention may have an excited state lifetime of not more than 150 $\mu$s, of not more than 100 $\mu$s, in particular of not more than 50 $\mu$s, more preferably of not more than 10 $\mu$s or not more than

7 μs in a film of poly(methyl methacrylate) (PMMA) with 10 % by weight of organic molecule at room temperature.

In some embodiments of the invention, the organic molecules according to the invention represent thermally-activated delayed fluorescence (TADF) emitters, which exhibit a $\Delta E_{ST}$ value, which corresponds to the energy difference between the first excited singlet state (S1) and the first excited triplet state (T1), of less than 5000 $cm^{-1}$, preferably less than 3000 $cm^{-1}$, more preferably less than 1500 $cm^{-1}$, even more preferably less than 1000 $cm^{-1}$ or even less than 500 $cm^{-1}$.

In a further embodiment of the invention, the energy level of the highest occupied molecular orbital HOMO(E) of the organic molecule according to the invention E ($E^{HOMO}$(E)) is larger than -6.2 eV, preferably larger than -6.0 eV, more preferably larger than -5.9 eV, even more preferably larger than -5.8 eV, wherein $E^{HOMO}$(E) is determined by cyclic voltammetry.

In a further embodiment of the invention, the energy level of the highest occupied molecular orbital HOMO(E) of the organic molecule according to the invention E ($E^{HOMO}$(E)) is larger than -5.8 eV, wherein $E^{HOMO}$(E) is determined by cyclic voltammetry.

In a further embodiment of the invention, the organic molecules according to the invention have an emission peak in the visible or nearest ultraviolet range, i.e., in the range of a wavelength of from 380 to 800 nm, with a full width at half maximum of less than 0.50 eV, preferably less than 0.48 eV, more preferably less than 0.45 eV, even more preferably less than 0.43 eV or even less than 0.40 eV in a film of poly(methyl methacrylate) (PMMA) with 10 % by weight of organic molecule at room temperature.

In a further embodiment of the invention, the organic molecules according to the invention have a "blue material index" (BMI), calculated by dividing the photoluminescence quantum yield (PLQY) in % by the CIEy color coordinate of the emitted light, of more than 150, in particular more than 200, preferably more than 250, more preferably of more than 300 or even more than 500. The photoluminescence quantum yield determination method applied is described in the experimental section.

Orbital and excited state energies can be determined either by means of experimental methods or by calculations employing quantum-chemical methods, in particular density functional theory calculations. The energy of the highest occupied molecular orbital $E^{HOMO}$ is determined by methods known to the person skilled in the art from cyclic voltammetry measurements with an accuracy of 0.1 eV. The cyclic voltammetry measurement methods are described in the experimental section. The energy of the lowest unoccupied molecular orbital $E^{LUMO}$ is calculated as $E^{HOMO}$ + $E^{gap}$, wherein $E^{gap}$ is determined as follows: For host compounds, the onset of the emission spectrum of a film with 10% by weight of host in poly(methyl methacrylate) (PMMA) is used as $E^{gap}$, unless stated otherwise. For emitter molecules, $E^{gap}$ is determined as the energy at which the excitation and emission spectra of a film with 10% by weight of emitter in PMMA cross.

The energy of the first excited triplet state T1 is determined from the onset of the emission spectrum at low temperature, typically at 77 K. For host compounds, where the first excited singlet state and the lowest triplet state are energetically separated by > 0.4 eV, the phosphorescence is usually visible in a steady-state spectrum in 2-Me-THF. The triplet energy can thus be determined as the onset of the phosphorescence spectrum. For TADF emitter molecules, the energy of the first excited triplet state T1 is determined from the onset of the delayed emission spectrum at 77 K, if not otherwise stated measured in a film of PMMA with 10% by weight of emitter. Both for host and emitter compounds, the energy of the first excited singlet state S1 is determined from the onset of the emission spectrum, if not otherwise stated measured in a film of PMMA with 10% by weight of host or emitter compound.

The onset of an emission spectrum is determined by computing the intersection of the tangent to the emission spectrum with the x-axis. The tangent to the emission spectrum is set at the high-energy side of the emission band and at the point at half maximum of the maximum intensity of the emission spectrum.

A further aspect of the invention relates to a process for preparing organic molecules (optionally with at least one subsequent reaction) according to the invention, wherein cyanuric chloride is used as a reactant:

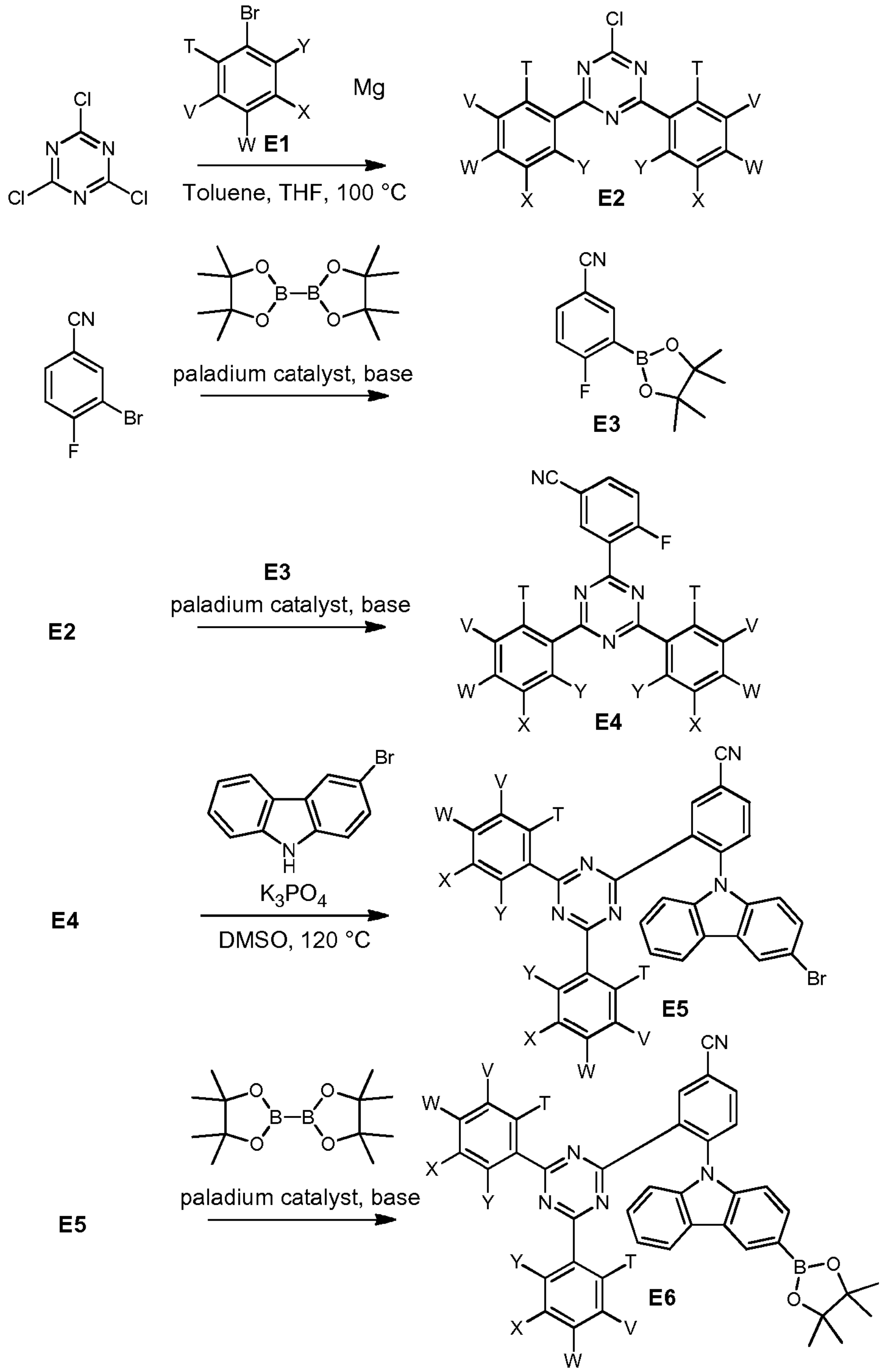
E1
Mg
Toluene, THF, 100 °C
E2
paladium catalyst, base
E3
E2
E3
paladium catalyst, base
E4
E4
$K_3PO_4$
DMSO, 120 °C
E5
E5
paladium catalyst, base
E6

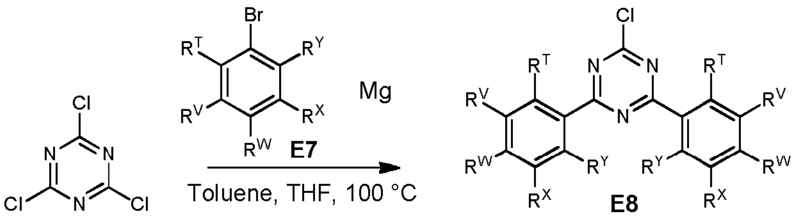


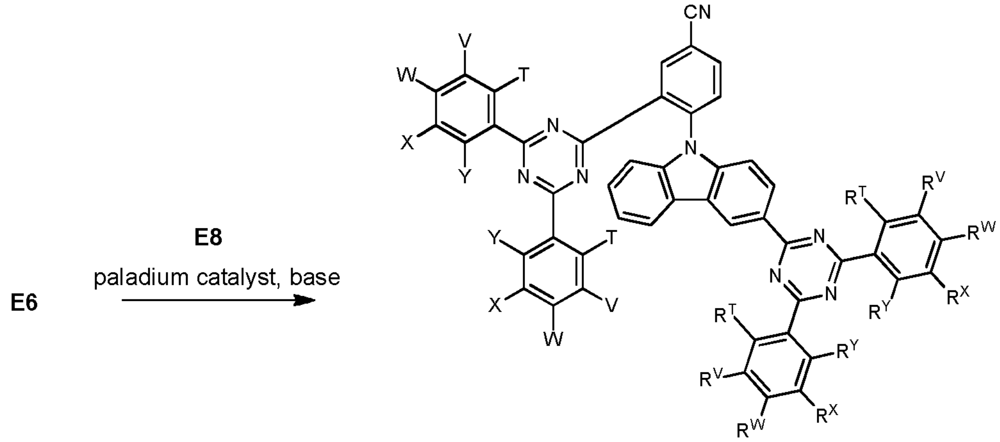


Alternatively, organic molecules according to the invention can be synthesized as follows:

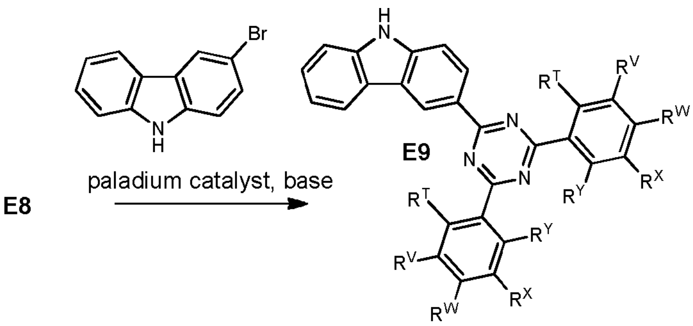


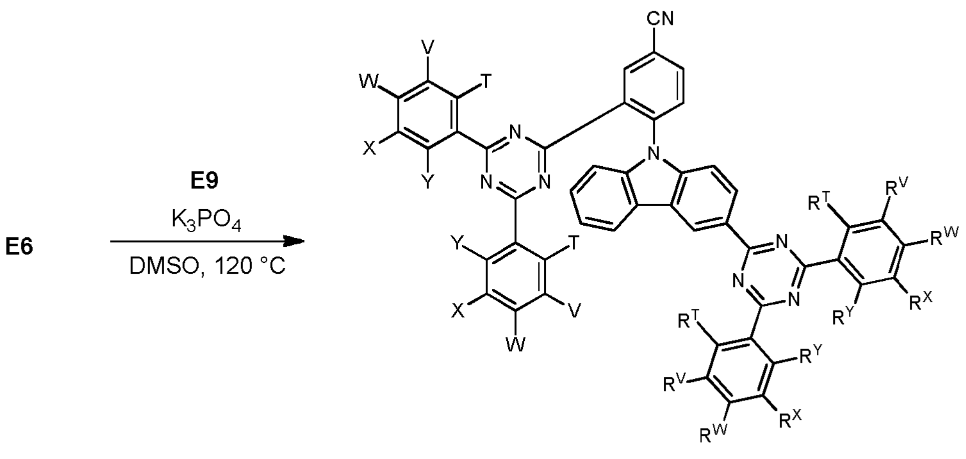


According to the invention, exactly one of the reactants selected from the group consisting of **E2** and **E8** might be 2-chloro-4,6-diphenyltriazine.

An alternative synthesis route comprises the introduction of a nitrogen heterocycle via copper- or palladium-catalyzed coupling to an aryl halide or aryl pseudohalide, preferably an aryl bromide, an aryl iodide, aryl triflate or an aryl tosylate.

Typically, $Pd_2(dba)_3$ (tris(dibenzylideneacetone)dipalladium(0)) is used as a Pd catalyst, but alternatives are

known in the art. For example, the ligand may be selected from the group consisting of S-Phos ([2-dicyclohexylphoshino-2',6'-dimethoxy-1,1'-biphenyl]), X-Phos (2-(dicyclohexylphosphino)-2",4",6"-triisopropylbiphenyl), and $P(Cy)_3$ (tricyclohexylphosphine). The salt is, for example, selected from tribasic potassium phosphate and potassium acetate and the solvent can be a pure solvent, such as toluene or dioxane, or a mixture, such as toluene/dioxane/water or dioxane/toluene. A person of skill in the art can determine which Pd catalyst, ligand, salt and solvent combination will result in high reaction yields.

For the reaction of a nitrogen heterocycle in a nucleophilic aromatic substitution with an aryl halide, preferably an aryl fluoride, typical conditions include the use of a base, such as tribasic potassium phosphate or sodium hydride, for example, in an aprotic polar solvent, such as dimethyl sulfoxide (DMSO) or N,N-dimethylformamide (DMF), for example.

A further aspect of the invention relates to the use of an organic molecule according to the invention as a luminescent emitter or as an absorber, and/or as a host material and/or as an electron transport material, and/or as a hole injection material, and/or as hole blocking material in an optoelectronic device, in particular as a luminescent emitter.

The optoelectronic device may be understood in the broadest sense as any device based on organic materials that is suitable for emitting light in the visible or nearest ultraviolet (UV) range, i.e., in the range of a wavelength of from 380 to 800 nm. More preferably, the optoelectronic device may be able to emit light in the visible range, i.e., of from 400 to 800 nm.

In the context of such use, the optoelectronic device is more particularly selected from the group consisting of:

- organic light-emitting diodes (OLEDs),
- light-emitting electrochemical cells,
- OLED sensors, in particular in gas and vapor sensors not hermetically externally shielded,
- organic diodes,
- organic solar cells,
- organic transistors,
- organic field-effect transistors,
- organic lasers, and
- down-conversion elements.

In a preferred embodiment in the context of such use, the optoelectronic device is a device selected from the group consisting of an organic light emitting diode (OLED), a light emitting electrochemical cell (LEC), and a light-emitting transistor.

In the case of the use, the fraction of the organic molecule according to the invention in the emission layer in an optoelectronic device, more particularly in OLEDs, is 1 % to 99 % by weight, more particularly 5 % to 80 % by weight. In an alternative embodiment, the proportion of the organic molecule in the emission layer is 100 % by weight.

In one embodiment, the light-emitting layer comprises not only the organic molecules according to the invention but also a host material whose triplet (T1) and singlet (S1) energy levels are energetically higher than the triplet (T1) and singlet (S1) energy levels of the organic molecule.

A further aspect of the invention relates to a composition comprising or consisting of:

(a) at least one organic molecule according to the invention, in particular in the form of an emitter and/or a host, and
(b) one or more emitter and/or host materials, which differ from the organic molecule according to the invention and
(c) optional one or more dyes and/or one or more solvents.

The one or more emitter and/or host which differ from the organic molecule according to the invention may comprise or consist of a polycyclic aromatic compound according to MAT2 or a specific example described in US-A 2015/236274. US-A 2015/236274 also describes examples for synthesis of such compounds.

In one embodiment, the one or more emitter and/or host which differfrom the organic molecule according to the invention comprise or consist of a structure according to Formula DAB-I:

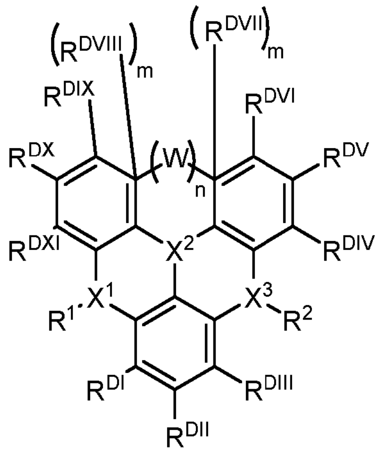

Formula DAB-I

wherein

n is 0 or 1.
m = 1-n.
$X^1$ is N or B.
$X^2$ is N or B.
$X^3$ is N or B.
W is selected from the group consisting of $Si(R^3)_2$, $C(R^3)_2$ and $BR^3$.
each of $R^1$, $R^2$ and $R^3$ is independently from each other selected from the group consisting of: $C_1$-$C_5$-alkyl, which is optionally substituted with one or more substituents $R^6$;
$C_6$-$C_{60}$-aryl, which is optionally substituted with one or more substituents $R^6$; and
$C_3$-$C_{57}$-heteroaryl, which is optionally substituted with one or more substituents $R^6$;
each of $R^{DI}$, $R^{DII}$, $R^{DIII}$, $R^{DIV}$, $R^{DV}$, $R^{DVI}$, $R^{DVII}$, $R^{DVIII}$ , $R^{DIX}$, $R^{DX}$, and $R^{DXI}$ is independently from another selected from the group consisting of: hydrogen, deuterium, $N(R^5)_2$, $OR^5$, $Si(R^5)_3$, $B(OR^5)_2$, $OSO_2R^5$, $CF_3$, CN, halogen, $C_1$-$C_{40}$-alkyl, which is optionally substituted with one or more substituents $R^5$ and wherein one or more non-adjacent $CH_2$-groups are each optionally substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$; $C_1$-$C_{40}$-alkoxy, which is optionally substituted with one or more substituents $R^5$ and wherein one or more non-adjacent $CH_2$-groups are each optionally substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$; $C_1$-$C_{40}$-thioalkoxy, which is optionally substituted with one or more substituents $R^5$ and wherein one or more non-adjacent $CH_2$-groups are each optionally substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$; $C_2$-$C_{40}$-alkenyl, which is optionally substituted with one or more substituents $R^5$ and wherein one or more non-adjacent $CH_2$-groups are each optionally substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$; $C_2$-$C_{40}$-alkynyl, which is optionally substituted with one or more substituents $R^5$ and wherein one or more non-adjacent $CH_2$-groups are each optionally substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$; $C_6$-$C_{60}$-aryl, which is optionally substituted with one or more substituents $R^5$; and
$C_3$-$C_{57}$-heteroaryl, which is optionally substituted with one or more substituents $R^5$.

$R^5$ is at each occurrence independently from another selected from the group consisting of: hydrogen, deuterium, OPh (Ph = phenyl), $CF_3$, CN, F,

$C_1$-$C_5$-alkyl, wherein optionally one or more hydrogen atoms are independently from each other substituted by deuterium, CN, $CF_3$, or F;
$C_1$-$C_5$-alkoxy, wherein optionally one or more hydrogen atoms are independently from each other substituted by deuterium, CN, $CF_3$, or F;
$C_1$-$C_5$-thioalkoxy, wherein optionally one or more hydrogen atoms are independently from each other substituted by deuterium, CN, $CF_3$, or F;
$C_2$-$C_5$-alkenyl, wherein optionally one or more hydrogen atoms are independently from each other substituted by deuterium, CN, $CF_3$, or F;

$C_2$-$C_5$-alkynyl, wherein optionally one or more hydrogen atoms are independently from each other substituted by deuterium, CN, $CF_3$, or F;
$C_6$-$C_{18}$-aryl, which is optionally substituted with one or more $C_1$-$C_5$-alkyl substituents; $C_3$-$C_{17}$-heteroaryl, which is optionally substituted with one or more $C_1$-$C_5$-alkyl substituents; N($C_6$-$C_{18}$-aryl)$_2$,
N($C_3$-$C_{17}$-heteroaryl)$_2$; and
N($C_3$-$C_{17}$-heteroaryl)($C_6$-$C_{18}$-aryl).

$R^6$ is at each occurrence independently from another selected from the group consisting of hydrogen, deuterium, OPh, $CF_3$, CN, F,

$C_1$-$C_5$-alkyl, wherein optionally one or more hydrogen atoms are independently from each other substituted by deuterium, CN, $CF_3$, or F;
$C_1$-$C_5$-alkoxy, wherein optionally one or more hydrogen atoms are independently from each other substituted by deuterium, CN, $CF_3$, or F;
$C_1$-$C_5$-thioalkoxy, wherein optionally one or more hydrogen atoms are independently from each other substituted by deuterium, CN, $CF_3$, or F;
$C_2$-$C_5$-alkenyl, wherein optionally one or more hydrogen atoms are independently from each other substituted by deuterium, CN, $CF_3$, or F;
$C_2$-$C_5$-alkynyl, wherein optionally one or more hydrogen atoms are independently from each other substituted by deuterium, CN, $CF_3$, or F;
$C_6$-$C_{18}$-aryl, which is optionally substituted with one or more $C_1$-$C_5$-alkyl substituents; $C_3$-$C_{17}$-heteroaryl, which is optionally substituted with one or more $C_1$-$C_5$-alkyl substituents; N($C_6$-$C_{18}$-aryl)$_2$,
N($C_3$-$C_{17}$-heteroaryl)$_2$; and
N($C_3$-$C_{17}$-heteroaryl)($C_6$-$C_{18}$-aryl).

According to a preferred embodiment, two or more of the substituents selected from the group consisting of $R^{DI}$, $R^{DII}$, $R^{DIII}$, $R^{DIV}$, $R^{DV}$, $R^{DVI}$, $R^{DVII}$, $R^{DVIII}$, $R^{DIX}$, $R^{DX}$, and $R^{DXI}$ that are positioned adjacent to another may each form a mono- or polycyclic, aliphatic, aromatic and/or benzo-fused ring system with another.

According to a preferred embodiment, at least one of $X^1$, $X^2$ and $X^3$ is B and at least one of $X^1$, $X^2$ and $X^3$ is N.

According to a preferred embodiment of the invention, at least one substituent selected from the group consisting of $R^{DI}$, $R^{DII}$, $R^{DIII}$, $R^{DIV}$, $R^{DV}$, $R^{DVI}$, $R^{DVII}$, $R^{DVIII}$, $R^{DIX}$, $R^{DX}$, and $R^{DXI}$ optionally forms a mono- or polycyclic, aliphatic, aromatic and/or benzo-fused ring system with one or more substituents of the same group that is/are positioned adjacent to the at least one substituent.

According to a preferred embodiment of the invention, at least one of $X^1$, $X^2$ and $X^3$ is B and at least one of $X^1$, $X^2$ and $X^3$ is N.

In one embodiment, the one or more emitter and/or host which differfrom the organic molecule according to the invention comprise or consist of a structure according to Formula DAB-I in which $X^1$ and $X^3$ each are N and $X^2$ is B, yielding Formula DAB-Ia:

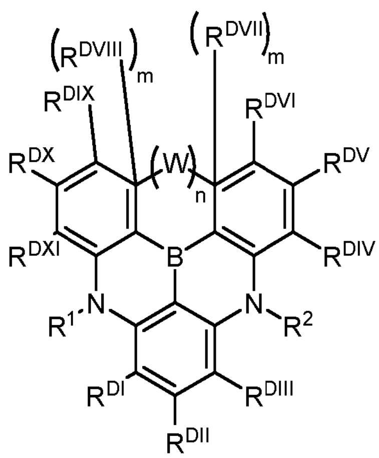

Formula DAB-Ia.

In one embodiment, the one or more emitter and/or host which differfrom the organic molecule according to

the invention comprise or consist of a structure according to Formula DAB-I in which $X^1$ and $X^3$ each are B and $X^2$ is N, yielding Formula DAB-Ib:

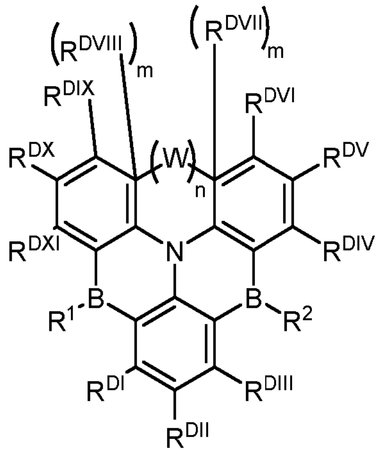

Formula DAB-Ib.

In one embodiment, the one or more emitter and/or host which differfrom the organic molecule according to the invention comprises or consists of a structure according to Formula DAB-I, DAB-Ia or DAB-Ib.

In one embodiment, each of $R^1$ and $R^2$ in Formulas DAB-I, DAB-Ia or DAB-Ib is each independently from each other selected from the group consisting of

$C_1$-$C_5$-alkyl, which is optionally substituted with one or more substituents $R^6$;
$C_6$-$C_{30}$-aryl, which is optionally substituted with one or more substituents $R^6$; and
$C_3$-$C_{30}$-heteroaryl, which is optionally substituted with one or more substituents $R^6$.

In one embodiment, $R^1$ and $R^2$ in Formulas DAB-I, DAB-Ia or DAB-Ib is each independently from each other selected from the group consisting of Me, $^i$Pr, $^t$Bu, CN, $CF_3$,

Ph, which is optionally substituted with one or more substituents independently from each other selected from the group consisting of Me, $^i$Pr, $^t$Bu, CN, $CF_3$, and Ph;
pyridinyl, which is optionally substituted with one or more substituents independently from each other selected from the group consisting of Me, $^i$Pr, $^t$Bu, CN, $CF_3$, and Ph;
pyrimidinyl, which is optionally substituted with one or more substituents independently from each other selected from the group consisting of Me, $^i$Pr, $^t$Bu, CN, $CF_3$, and Ph; and
triazinyl, which is optionally substituted with one or more substituents independently from each other selected from the group consisting of Me, $^i$Pr, $^t$Bu, CN, $CF_3$, and Ph.

In one embodiment, each of $R^{DI}$, $R^{DII}$, $R^{DIII}$, $R^{DIV}$, $R^{DV}$, $R^{DVI}$, $R^{DVII}$, $R^{DVIII}$, $R^{DIX}$, $R^{DX}$, and $R^{DXI}$ in Formulas DAB-I, DAB-Ia or DAB-Ib is independently from another selected from the group consisting of: hydrogen, deuterium, halogen, Me, $^i$Pr, $^t$Bu, CN, $CF_3$,

Ph, which is optionally substituted with one or more substituents independently from each other selected from the group consisting of Me, $^i$Pr, $^t$Bu, CN, $CF_3$, and Ph;
pyridinyl, which is optionally substituted with one or more substituents independently from each other selected from the group consisting of Me, $^i$Pr, $^t$Bu, CN, $CF_3$, and Ph;
pyrimidinyl, which is optionally substituted with one or more substituents independently from each other selected from the group consisting of Me, $^i$Pr, $^t$Bu, CN, $CF_3$, and Ph;
carbazolyl, which is optionally substituted with one or more substituents independently from each other selected from the group consisting of Me, $^i$Pr, $^t$Bu, CN, $CF_3$, and Ph;
triazinyl, which is optionally substituted with one or more substituents independently from each other selected from the group consisting of Me, $^i$Pr, $^t$Bu, CN, $CF_3$, and Ph;

and $N(Ph)_2$.

In one embodiment, each of $R^{DI}$, $R^{DII}$, $R^{DIII}$, $R^{DIV}$, $R^{DV}$, $R^{DVI}$, $R^{DVII}$, $R^{DVIII}$, $R^{DIX}$, $R^{DX}$, and $R^{DXI}$ in Formulas DAB-I, DAB-Ia or DAB-Ib is independently from another selected from the group consisting of: hydrogen, deuterium, halogen, Me, $^{i}$Pr, $^{t}$Bu, CN, $CF_3$,

Ph, which is optionally substituted with one or more substituents independently from each other selected from the group consisting of Me, $^{i}$Pr, $^{t}$Bu, CN, $CF_3$, and Ph;
pyridinyl, which is optionally substituted with one or more substituents independently from each other selected from the group consisting of Me, $^{i}$Pr, $^{t}$Bu, CN, $CF_3$, and Ph;
pyrimidinyl, which is optionally substituted with one or more substituents independently from each other selected from the group consisting of Me, $^{i}$Pr, $^{t}$Bu, CN, $CF_3$, and Ph;
carbazolyl, which is optionally substituted with one or more substituents independently from each other selected from the group consisting of Me, $^{i}$Pr, $^{t}$Bu, CN, $CF_3$, and Ph;
triazinyl, which is optionally substituted with one or more substituents independently from each other selected from the group consisting of Me, $^{i}$Pr, $^{t}$Bu, CN, $CF_3$, and Ph;
and $N(Ph)_2$; and
$R^1$ and $R^2$ is each independently from each other selected from the group consisting of $C_1$-$C_5$-alkyl, which is optionally substituted with one or more substituents $R^6$;
$C_6$-$C_{30}$-aryl, which is optionally substituted with one or more substituents $R^6$; and
$C_3$-$C_{30}$-heteroaryl, which is optionally substituted with one or more substituents $R^6$.

In one embodiment, the light-emitting layer EML comprises or consists of a composition comprising or consisting of:

(a) at least one organic molecule according to the invention, in particular in the form of an emitter and/or a host, and
(b) one or more emitter and/or host materials, which differ from the organic molecule according to the invention and
(c) optional one or more dyes and/or one or more solvents.

Particularly preferably the light-emitting layer EML comprises or) consists of a composition comprising or consisting of:

(i) 1-50 % by weight, preferably 5-40 % by weight, in particular 10-30 % by weight, of one or more organic molecules according to the invention E;
(ii) 5-99 % by weight, preferably 30-94.9 % by weight, in particular 40-89% by weight, of at least one host compound H; and
(iii) optionally 0-94 % by weight, preferably 0.1-65 % by weight, in particular 1-50 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) optionally 0-30 % by weight, in particular 0-20 % by weight, preferably 0-5 % by weight, of at least one further emitter molecule F with a structure differing from the structure of the molecules according to the invention.

Preferably, energy can be transferred from the host compound H to the one or more organic molecules according to the invention E, in particular transferred from the first excited triplet state T1(H) of the host compound H to the first excited triplet state T1(E) of the one or more organic molecules according to the invention E and/ orfrom the first excited singlet state S1(H) of the host compound H to the first excited singlet state S1(E) of the one or more organic molecules according to the invention E.

In a further embodiment, the light-emitting layer EML comprises or (essentially) consists of a composition comprising or consisting of:

(i) 1-50 % by weight, preferably 5-40 % by weight, in particular 10-30 % by weight, of one organic molecule according to the invention E;
(ii) 5-99 % by weight, preferably 30-94.9 % by weight, in particular 40-89% by weight, of one host compound H; and
(iii) optionally, 0-94 % by weight, preferably 0.1-65 % by weight, in particular 1-50 % by weight, of at least one further host compound D with a structure differing from the structure of the molecules according to the invention; and
(iv) optionally, 0-94 % by weight, preferably 0-65 % by weight, in particular 0-50 % by weight, of a solvent; and
(v) optionally, 0-30 % by weight, in particular 0-20 % by weight, preferably 0-5 % by weight, of at least one further emitter molecule F with a structure differing from the structure of the molecules according to the invention.

In one embodiment, the host compound H has a highest occupied molecular orbital HOMO(H) having an energy

$E^{HOMO}(H)$ in the range of from -5 to -6.5 eV and the at least one further host compound D has a highest occupied molecular orbital HOMO(D) having an energy $E^{HOMO}(D)$, wherein $E^{HOMO}(H) > E^{HOMO}(D)$.

In further embodiments, the host compound H has a lowest unoccupied molecular orbital LUMO(H) having an energy $E^{LUMO}(H)$ and the at least one further host compound D has a lowest unoccupied molecular orbital LUMO(D) having an energy $E^{LUMO}(D)$, wherein $E^{LUMO}(H) > E^{LUMO}(D)$.

In some embodiments, the host compound H has a highest occupied molecular orbital HOMO(H) having an energy $E^{HOMO}(H)$ and a lowest unoccupied molecular orbital LUMO(H) having an energy $E^{LUMO}(H)$, and

the at least one further host compound D has a highest occupied molecular orbital HOMO(D) having an energy $E^{HOMO}(D)$ and a lowest unoccupied molecular orbital LUMO(D) having an energy $E^{LUMO}(D)$,

the organic molecule according to the invention E has a highest occupied molecular orbital HOMO(E) having an energy $E^{HOMO}(E)$ and a lowest unoccupied molecular orbital LUMO(E) having an energy $E^{LUMO}(E)$,

wherein

$E^{HOMO}(H) > E^{HOMO}(D)$ and the difference between the energy level of the highest occupied molecular orbital HOMO(E) of the organic molecule according to the invention E ($E^{HOMO}(E)$) and the energy level of the highest occupied molecular orbital HOMO(H) of the host compound H ($E^{HOMO}(H)$) is between -0.5 eV and 0.5 eV, more preferably between -0.3 eV and 0.3 eV, even more preferably between -0.2 eV and 0.2 eV or even between -0.1 eV and 0.1 eV; and $E^{LUMO}(H) > E^{LUMO}(D)$ and the difference between the energy level of the lowest unoccupied molecular orbital LUMO(E) of the organic molecule according to the invention E ($E^{LUMO}(E)$) and the lowest unoccupied molecular orbital LUMO(D) of the at least one further host compound D ($E^{LUMO}(D)$) is between -0.5 eV and 0.5 eV, more preferably between -0.3 eV and 0.3 eV, even more preferably between -0.2 eV and 0.2 eV or even between -0.1 eV and 0.1 eV.

In a further aspect, the invention relates to an optoelectronic device comprising an organic molecule or a composition of the type described here, more particularly in the form of a device selected from the group consisting of organic light-emitting diode (OLED), light-emitting electrochemical cell, OLED sensor, more particularly gas and vapor sensors not hermetically externally shielded, organic diode, organic solar cell, organic transistor, organic field-effect transistor, organic laser and down-conversion element.

In a preferred embodiment, the optoelectronic device is a device selected from the group consisting of an organic light emitting diode (OLED), a light emitting electrochemical cell (LEC), and a light-emitting transistor.

In one embodiment of the optoelectronic device of the invention, the organic molecule according to the invention E is used as emission material in a light-emitting layer EML.

In one embodiment of the optoelectronic device of the invention the light-emitting layer EML consists of the composition according to the invention described here.

For example, when the optoelectronic device is an OLED, it may exhibit the following layer structure:

1. substrate
2. anode layer A
3. hole injection layer, HIL
4. hole transport layer, HTL
5. electron blocking layer, EBL
6. emitting layer, EML
7. hole blocking layer, HBL
8. electron transport layer, ETL
9. electron injection layer, EIL
10. cathode layer,

wherein the OLED comprises each layer only optionally, except for the EML, which is mandatory. In some embodiments, different layers may be merged and the OLED may comprise more than one layer of each layer type as defined above.

Furthermore, the optoelectronic device may optionally comprise one or more protective layers protecting the device from damaging exposure to harmful species in the environment including, exemplarily moisture, vapor and/or gases.

In one embodiment of the invention, the optoelectronic device is an OLED, which exhibits the following inverted layer structure:

1. substrate
2. cathode layer
3. electron injection layer, EIL
4. electron transport layer, ETL

5. hole blocking layer, HBL
6. emitting layer, B
7. electron blocking layer, EBL
8. hole transport layer, HTL
9. hole injection layer, HIL
10. anode layer A

wherein the OLED with an inverted layer structure comprises each layer other than the emitting layer B only optionally. In some embodiments, different layers may be merged and the OLED may comprise more than one layer of each layer types defined above.

In one embodiment of the invention, the optoelectronic device is an OLED, which may exhibit stacked architecture. In this architecture, contrary to the typical arrangement, where the OLEDs are placed side by side, the individual units are stacked on top of each other. Blended light may be generated with OLEDs exhibiting a stacked architecture, in particular white light may be generated by stacking blue, green and red OLEDs. Furthermore, the OLED exhibiting a stacked architecture may optionally comprise a charge generation layer (CGL), which is typically located between two OLED subunits and typically consists of a n-doped and p-doped layer with the n-doped layer of one CGL being typically located closer to the anode layer.

In one embodiment of the invention, the optoelectronic device is an OLED, which comprises two or more emission layers between anode and cathode. In particular, this so-called tandem OLED comprises three emission layers, wherein one emission layer emits red light, one emission layer emits green light and one emission layer emits blue light, and optionally may comprise further layers such as charge generation layers, blocking or transporting layers between the individual emission layers. In a further embodiment, the emission layers are adjacently stacked. In a further embodiment, the tandem OLED comprises a charge generation layer between each two emission layers. In addition, adjacent emission layers or emission layers separated by a charge generation layer may be merged.

The substrate may be formed by any material or composition of materials. Most frequently, glass slides are used as substrates. Alternatively, thin metal layers (e.g., copper, gold, silver or aluminum films) or plastic films or slides may be used. This may allow a higher degree of flexibility. The anode layer A is mostly composed of materials allowing to obtain an (essentially) transparent film. As at least one of both electrodes should be (essentially) transparent in order to allow light emission from the OLED, either the anode layer A or the cathode layer C is transparent. Preferably, the anode layer A comprises a large content or even consists of transparent conductive oxides (TCOs). Such anode layer A may exemplarily comprise indium tin oxide, aluminum zinc oxide, fluorine doped tin oxide, indium zinc oxide, PbO, SnO, zirconium oxide, molybdenum oxide, vanadium oxide, wolfram oxide, graphite, doped Si, doped Ge, doped GaAs, doped polyaniline, doped polypyrrol and/or doped polythiophene.

Particularly preferably, the anode layer A (essentially) consists of indium tin oxide (ITO) (e.g., $(InO_3)0.9(SnO_2)0.1$). The roughness of the anode layer A caused by the transparent conductive oxides (TCOs) may be compensated by using a hole injection layer (HIL). Further, the HIL may facilitate the injection of quasi charge carriers (i.e., holes) in that the transport of the quasi charge carriers from the TCO to the hole transport layer (HTL) is facilitated. The hole injection layer (HIL) may comprise poly-3,4-ethylendioxy thiophene (PEDOT), polystyrene sulfonate (PSS), $MoO_2$, $V_2O_5$, CuPC or CuI, in particular a mixture of PEDOT and PSS. The hole injection layer (HIL) may also prevent the diffusion of metals from the anode layer A into the hole transport layer (HTL). The HIL may exemplarily comprise PEDOT:PSS (poly-3,4-ethylendioxy thiophene: polystyrene sulfonate), PEDOT (poly-3,4-ethylendioxy thiophene), mMTDATA (4,4',4"-tris[phenyl(m-tolyl)amino]triphenylamine), Spiro-TAD (2,2',7,7'-tetrakis(n,n-diphenylamino)-9,9'-spirobifluorene), DNTPD (N1,N1'-(biphenyl-4,4'-diyl)bis(N1-phenyl-N4,N4-di-m-tolylbenzene-1,4-diamine), NPB (N,N'-nis-(1-naphthalenyl)-N,N'-bisphenyl-(1,1'-biphenyl)-4,4'-diamine), NPNPB (N,N'-diphenyl-N,N'-di-[4-(N,N-diphenyl-amino)phenyl]benzidine), MeO-TPD (N,N,N',N'-tetrakis(4-methoxyphenyl)benzidine), HAT-CN (1,4,5,8,9,11-hexaazatriphenylen-hexacarbonitrile) and/or Spiro-NPD (N,N'-diphenyl-N,N'-bis-(1-naphthyl)-9,9'-spirobifluorene-2,7-diamine).

Adjacent to the anode layer A or hole injection layer (HIL) typically a hole transport layer (HTL) is located. Herein, any hole transport compound may be used. Exemplarily, electron-rich heteroaromatic compounds such as triarylamines and/or carbazoles may be used as hole transport compound. The HTL may decrease the energy barrier between the anode layer A and the light-emitting layer EML. The hole transport layer (HTL) may also be an electron blocking layer (EBL). Preferably, hole transport compounds bear comparably high energy levels of their triplet states T1. Exemplarily the hole transport layer (HTL) may comprise a star-shaped heterocycle such as tris(4-carbazoyl-9-ylphenyl)amine (TCTA), poly-TPD (poly(4-butylphenyl-diphenyl-amine)), [alpha]-NPD (poly(4-butylphenyl-diphenyl-amine)), TAPC (4,4'-cyclohexyliden-bis[N,N-bis(4-methylphenyl)benzenamine]), 2-TNATA (4,4',4"-tris[2-naphthyl(phenyl)amino]triphenylamine), Spiro-TAD, DNTPD, NPB, NPNPB, MeO-TPD, HAT-CN and/or TrisPcz (9,9'-diphenyl-6-(9-phenyl-9H-carbazol-3-yl)-9H,9'H-3,3'-bicarbazole). In addition, the HTL may comprise a p-doped layer, which may be composed of an inorganic or organic dopant in an organic hole-transporting matrix. Transition metal oxides such as vanadium oxide, molybdenum oxide or tungsten oxide may exemplarily be used as inorganic dopant. Tetrafluorotetra-

cyanoquinodimethane ($F_4$-TCNQ), copper-pentafluorobenzoate (Cu(I)pFBz) or transition metal complexes may exemplarily be used as organic dopant.

The EBL may exemplarily comprise mCP (1,3-bis(carbazol-9-yl)benzene), TCTA, 2-TNATA, mCBP (3,3-di(9H-carbazol-9-yl)biphenyl), tris-Pcz, CzSi (9-(4-tert-Butylphenyl)-3,6-bis(triphenylsilyl)-9H-carbazole), and/or DCB (N,N'-dicarbazolyl-1,4-dimethylbenzene).

Adjacent to the hole transport layer (HTL), typically, the light-emitting layer EML is located. The light-emitting layer EML comprises at least one light emitting molecule. Particularly, the EML comprises at least one light emitting molecule according to the invention E. In one embodiment, the light-emitting layer comprises only the organic molecules according to the invention E. Typically, the EML additionally comprises one or more host materials H. Exemplarily, the host material H is selected from CBP (4,4'-Bis-(N-carbazolyl)-biphenyl), mCP, mCBP Sif87 (dibenzo[b,d]thiophen-2-yltriphenylsilane), CzSi, SiMCP (3,5-Di(9H-carbazol-9-yl)phenyl]triphenylsilane), Sif88 (dibenzo[b,d]thiophen-2-yl)diphenylsilane), DPEPO (bis[2-(diphenylphosphino)phenyl] ether oxide), 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole, 9-[3,5-bis(2-dibenzothiophenyl)phenyl]-9H-carbazole, T2T (2,4,6-tris(biphenyl-3-yl)-1,3,5-triazine), T3T (2,4,6-tris(triphenyl-3-yl)-1,3,5-triazine) and/or TST (2,4,6-tris(9,9'-spirobifluorene-2-yl)-1,3,5-triazine). The host material H typically should be selected to exhibit first triplet (T1) and first singlet (S1) energy levels, which are energetically higher than the first triplet (T1) and first singlet (S1) energy levels of the organic molecule.

In one embodiment of the invention, the EML comprises a so-called mixed-host system with at least one hole-dominant host and one electron-dominant host. In a particular embodiment, the EML comprises exactly one light emitting molecule according to the invention E and a mixed-host system comprising T2T as electron-dominant host and a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole as hole-dominant host. In a further embodiment the EML comprises 50-80 % by weight, preferably 60-75 % by weight of a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole; 10-45 % by weight, preferably 15-30 % by weight of T2T and 5-40 % by weight, preferably 10-30 % by weight of light emitting molecule according to the invention.

Adjacent to the light-emitting layer EML an electron transport layer (ETL) may be located. Herein, any electron transporter may be used. Exemplarily, electron-poor compounds such as, e.g., benzimidazoles, pyridines, triazoles, oxadiazoles (e.g., 1,3,4-oxadiazole), phosphinoxides and sulfone, may be used. An electron transporter may also be a star-shaped heterocycle such as 1,3,5-tri(1-phenyl-1H-benzo[d]imidazol-2-yl)phenyl (TPBi). The ETL may comprise NBphen (2,9-bis(naphthalen-2-yl)-4,7-diphenyl-1,10-phenanthroline), $Alq_3$ (Aluminum-tris(8-hydroxyquinoline)), TSPO1 (diphenyl-4-triphenylsilylphenyl-phosphinoxide), BPyTP2 (2,7-di(2,2'-bipyridin-5-yl)triphenyle), Sif87 (dibenzo[b,d]thiophen-2-yltriphenylsilane), Sif88 (dibenzo[b,d]thiophen-2-yl)diphenylsilane), BmPyPhB (1,3-bis[3,5-di(pyridin-3-yl)phenyl]benzene) and/or BTB (4,4'-bis-[2-(4,6-diphenyl-1,3,5-triazinyl)]-1,1'-biphenyl). Optionally, the ETL may be doped with materials such as Liq. The electron transport layer (ETL) may also block holes or a holeblocking layer (HBL) is introduced.

The HBL may, for example, comprise BCP (2,9-dimethyl-4,7-diphenyl-1,10-phenanthroline = Bathocuproine), BAlq (bis(8-hydroxy-2-methylquinoline)-(4-phenylphenoxy)aluminum), NBphen (2,9-bis(naphthalen-2-yl)-4,7-diphenyl-1,10-phenanthroline), $Alq_3$ (Aluminum-tris(8-hydroxyquinoline)), TSPO1 (diphenyl-4-triphenylsilylphenyl-phosphinoxide), T2T (2,4,6-tris(biphenyl-3-yl)-1,3,5-triazine), T3T (2,4,6-tris(triphenyl-3-yl)-1,3,5-triazine), TST (2,4,6-tris(9,9'-spirobifluorene-2-yl)-1,3,5-triazine), and/or TCB/TCP (1,3,5-tris(N-carbazolyl)benzol/ 1,3,5-tris(carbazol)-9-yl) benzene).

Adjacent to the electron transport layer (ETL), a cathode layer C may be located. Exemplarily, the cathode layer C may comprise or may consist of a metal (e.g., Al, Au, Ag, Pt, Cu, Zn, Ni, Fe, Pb, LiF, Ca, Ba, Mg, In, W, or Pd) or a metal alloy. For practical reasons, the cathode layer may also consist of (essentially) non-transparent metals such as Mg, Ca or Al. Alternatively or additionally, the cathode layer C may also comprise graphite and or carbon nanotubes (CNTs). Alternatively, the cathode layer C may also consist of nanoscalic silver wires.

An OLED may further, optionally, comprise a protection layer between the electron transport layer (ETL) and the cathode layer C (which may be designated as electron injection layer (EIL)). This layer may comprise lithium fluoride, cesium fluoride, silver, Liq (8-hydroxyquinolinolatolithium), $Li_2O$, $BaF_2$, MgO and/or NaF.

Optionally, also the electron transport layer (ETL) and/or a hole blocking layer (HBL) may comprise one or more host compounds H.

In order to modify the emission spectrum and/or the absorption spectrum of the light-emitting layer EML further, the light-emitting layer EML may further comprise one or more further emitter molecules F. Such an emitter molecule F may be any emitter molecule known in the art. Preferably such an emitter molecule F is a molecule with a structure differing from the structure of the molecules according to the invention E. The emitter molecule F may optionally be a

TADF emitter. Alternatively, the emitter molecule F may optionally be a fluorescent and/or phosphorescent emitter molecule which is able to shift the emission spectrum and/or the absorption spectrum of the light-emitting layer EML. Exemplarily, the triplet and/or singlet excitons may be transferred from the emitter molecule according to the invention E to the emitter molecule F before relaxing to the ground state S0 by emitting light typically red-shifted in comparison to the light emitted by emitter molecule E. Optionally, the emitter molecule F may also provoke two-photon effects (i.e., the absorption of two photons of half the energy of the absorption maximum).

Optionally, an optoelectronic device (e.g., an OLED) may exemplarily be an essentially white optoelectronic device. Exemplarily such white optoelectronic device may comprise at least one (deep) blue emitter molecule and one or more emitter molecules emitting green and/or red light. Then, there may also optionally be energy transmittance between two or more molecules as described above.

As used herein, if not defined more specifically in the particular context, the designation of the colors of emitted and/or absorbed light is as follows:

| | |
|---|---|
| violet: | wavelength range of >380-420 nm; |
| deep blue: | wavelength range of >420-480 nm; |
| sky blue: | wavelength range of >480-500 nm; |
| green: | wavelength range of >500-560 nm; |
| yellow: | wavelength range of >560-580 nm; |
| orange: | wavelength range of >580-620 nm; |
| red: | wavelength range of >620-800 nm |

With respect to emitter molecules, such colors refer to the emission maximum. Therefore, exemplarily, a deep blue emitter has an emission maximum in the range of from >420 to 480 nm, a sky blue emitter has an emission maximum in the range of from >480 to 500 nm, a green emitter has an emission maximum in a range of from >500 to 560 nm, a red emitter has an emission maximum in a range of from >620 to 800 nm.

A deep blue emitter may preferably have an emission maximum of below 480 nm, more preferably below 470 nm, even more preferably below 465 nm or even below 460 nm. It will typically be above 420 nm, preferably above 430 nm, more preferably above 440 nm or even above 450 nm.

Accordingly, a further aspect of the present invention relates to an OLED, which exhibits an external quantum efficiency at 1000 cd/m$^2$ of more than 8 %, more preferably of more than 10 %, more preferably of more than 13 %, even more preferably of more than 15 % or even more than 20 % and/or exhibits an emission maximum between 420 nm and 500 nm, preferably between 430 nm and 490 nm, more preferably between 440 nm and 480 nm, even more preferably between 450 nm and 470 nm and/or exhibits a LT80 value at 500 cd/m$^2$ of more than 100 h, preferably more than 200 h, more preferably more than 400 h, even more preferably more than 750 h or even more than 1000 h. Accordingly, a further aspect of the present invention relates to an OLED, whose emission exhibits a CIEy color coordinate of less than 0.45, preferably less than 0.30, more preferably less than 0.20 or even more preferably less than 0.15 or even less than 0.10.

A further aspect of the present invention relates to an OLED, which emits light at a distinct color point. According to the present invention, the OLED emits light with a narrow emission band (small full width at half maximum (FWHM)). In one aspect, the OLED according to the invention emits light with a FWHM of the main emission peak of less than 0.50 eV, preferably less than 0.48 eV, more preferably less than 0.45 eV, even more preferably less than 0.43 eV or even less than 0.40 eV.

A further aspect of the present invention relates to an OLED, which emits light with CIEx and CIEy color coordinates close to the CIEx and CIEy color coordinates of the primary color blue (CIEx = 0.131 and CIEy = 0.046) as defined by ITU-R Recommendation BT.2020 (Rec. 2020) and thus is suited for the use in Ultra High Definition (UHD) displays, e.g. UHD-TVs. Accordingly, a further aspect of the present invention relates to an OLED, whose emission exhibits a CIEx color coordinate of between 0.02 and 0.30, preferably between 0.03 and 0.25, more preferably between 0.05 and 0.20 or even more preferably between 0.08 and 0.18 or even between 0.10 and 0.15 and/ or a CIEy color coordinate of between 0.00 and 0.45, preferably between 0.01 and 0.30, more preferably between 0.02 and 0.20 or even more preferably between 0.03 and 0.15 or even between 0.04 and 0.10.

In a further aspect, the invention relates to a method for producing an optoelectronic component. In this case an organic molecule of the invention is used.

The optoelectronic device, in particular the OLED according to the present invention can be produced by any means of vapor deposition and/or liquid processing. Accordingly, at least one layer is

- prepared by means of a sublimation process,

- prepared by means of an organic vapor phase deposition process,
- prepared by means of a carrier gas sublimation process,
- solution processed, or
- printed.

Another aspect of the present invention relates to a process for producing an optoelectronic device, wherein an organic molecule or composition according to the invention is used, in particular comprising the processing of the organic molecule or of the composition by a vacuum evaporation method or from a solution.

Further steps used to produce the optoelectronic device, in particular the OLED according to the present invention, comprise depositing different layers individually and successively on a suitable substrate by means of subsequent deposition processes. The individual layers may be deposited using the same or differing deposition methods.

Vapor deposition processes exemplarily comprise thermal (co)evaporation, chemical vapor deposition and physical vapor deposition. For active matrix OLED display, an AMOLED backplane is used as substrate. The individual layer may be processed from solutions or dispersions employing adequate solvents. Solution deposition process exemplarily comprise spin coating, dip coating and jet printing. Liquid processing may optionally be carried out in an inert atmosphere (e.g., in a nitrogen atmosphere) and the solvent may optionally be completely or partially removed by means known in the state of the art.

**Examples**

General synthesis scheme I

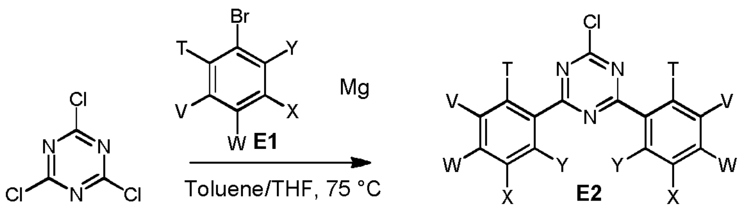


E2 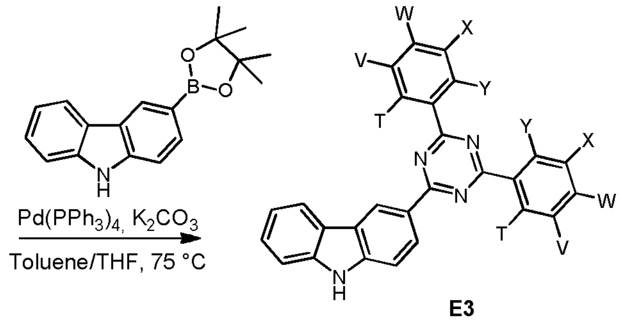


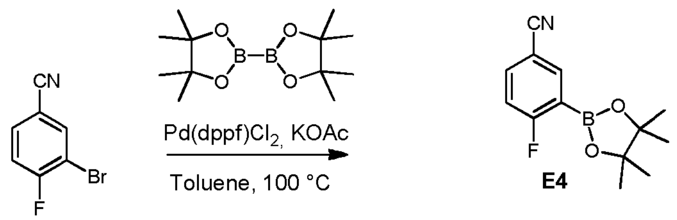


E2 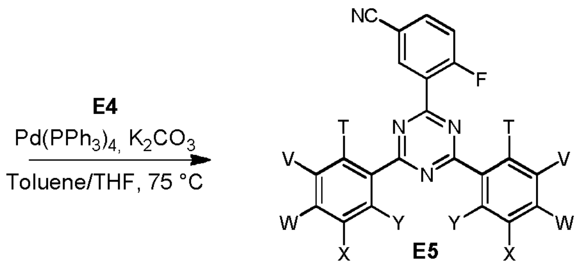


E5 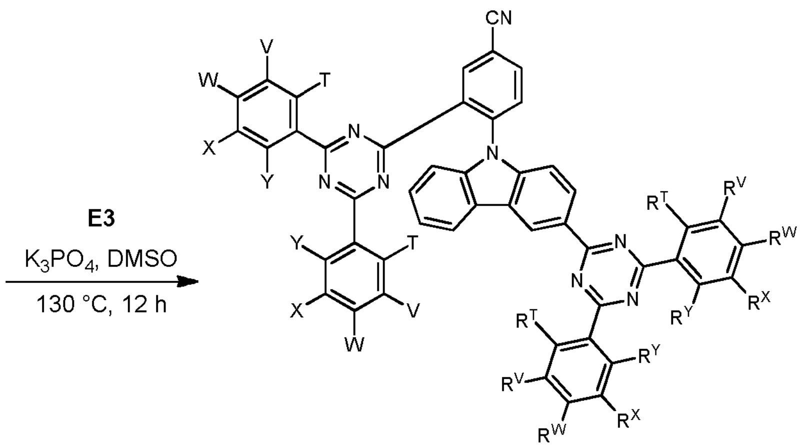


*General procedure for synthesis **AAV1***

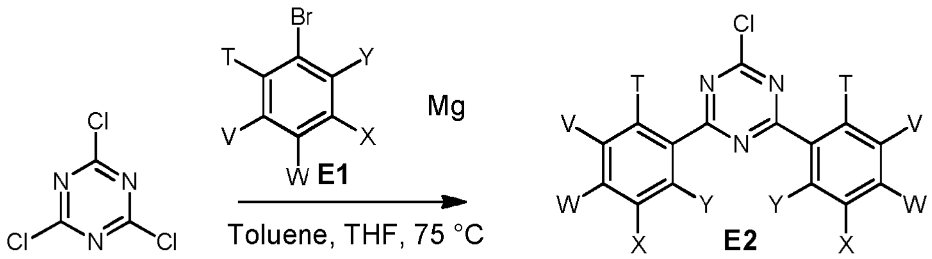


In a nitrogen atmosphere, a solution of **E1** (2.50 eq.) in dry THF was added dropwise to a mixture of Mg-turnings in dry THF. After the exothermic reaction, the reaction mixture was refluxed for 3 h and then cooled to room temperature. This Grignard solution was then slowly added to cyanuric chloride (1.00 eq.) in dry toluene. The reaction mixture was heated to 90 °C for 30 min. Reaction progress / completion of the reaction was checked using GCMS. After completion of the reaction, the reaction mixture was quenched with hydrochloric acid (1 mol/l) and afterwards extracted with dichloromethane, washed with brine and dried over magnesium sulfate. Crude product was purified by recrystallization from n-hexane to yield **E2**.

*General procedure for synthesis **AAV2***

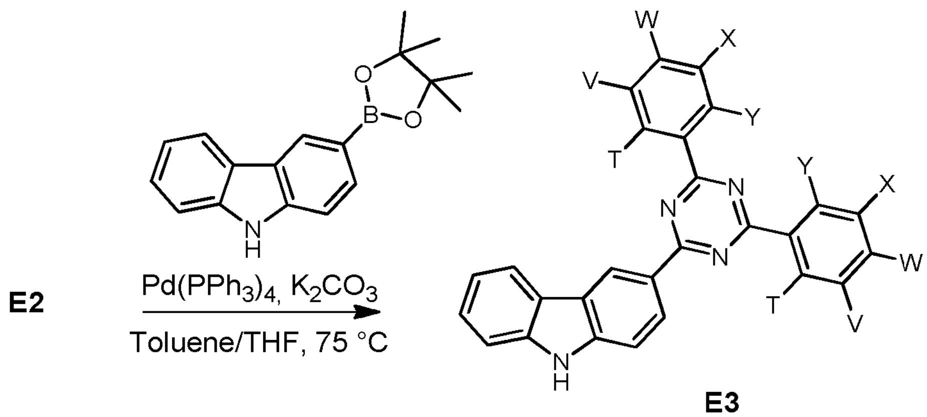


**E2** (1.00 equivalent), 3-(4,4,5,5-tetramethyl-1,3,2-dioxaborolan-2-yl)carbazole (CAS 855738-89-5) (1.1 equivalents), $Pd(PPh_3)_4$ (0.01 equivalents, CAS 14221-01-3), and potassium carbonate (2 equivalents, 584-08-7), are stirred under nitrogen atmosphere in a toluene/THF mixture (ratio: 10:1) at 75 °C overnight. After cooling down to room temperature (RT), the reaction mixture is extracted with dichloromethane and washed with water. The organic layer is dried over MgSO4, filtered and concentrated. The crude product is purified by flash chromatography to yield **E3**.

*General procedure for synthesis **AAV3***

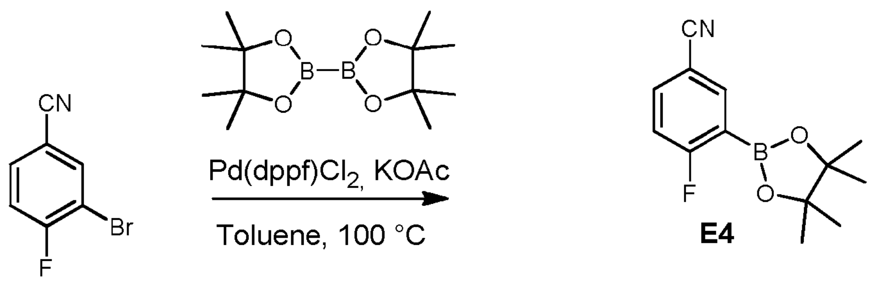

3-bromo-4-fluorobenzonitrile (1.00 equivalent CAS 79630-23-2), bis-(pinacolato)diboron (1.5 equivalents, CAS 73183-34-3), [1,1'-bis(diphenylphosphino)ferrocene]palladium (II) dichloride (Pd(dppf)$Cl_2$, 0.02 equivalents, 72287-26-4), and potassium acetate (KOAc, 3.0 equivalents) are stirred under nitrogen atmosphere in dry toluene at 110 °C for 16 h. After cooling down to room temperature (RT), the reaction mixture is extracted with ethyl acetate. The organic phases are collected, washed with brine and dried over $MgSO_4$. The organic solvent is removed, the crude product was washed with cyclohexane and recrystallized from EtOH to yield **E4.**

*General procedure for synthesis **AAV4***

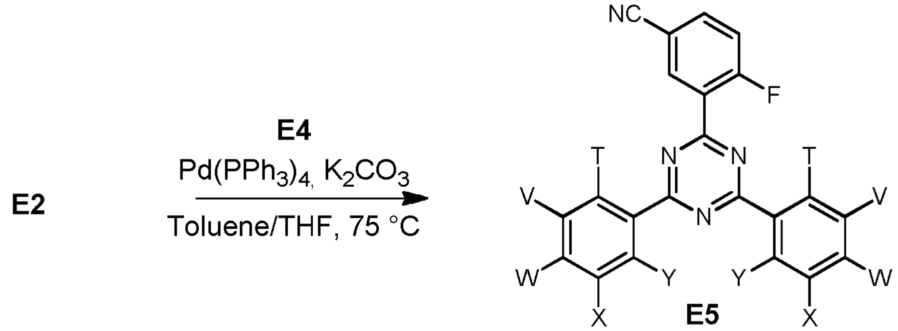


**E2** (1.00 equivalent), **E4** (1.50 equivalents), Pd($PPh_3$)$_4$ (0.01 equivalents), potassium carbonate (2.00 equivalents) are stirred under nitrogen atmosphere in a toluene/THF mixture (ratio: 10:1) at 75 °C overnight. After cooling down to room temperature (RT), the reaction mixture is extracted with ethyl acetate and washed with brine. The organic layer is dried over MgSO4, filtered and concentrated. The crude product is purified by flash chromatography to yield **E5**.

*General procedure for synthesis **AAV5***

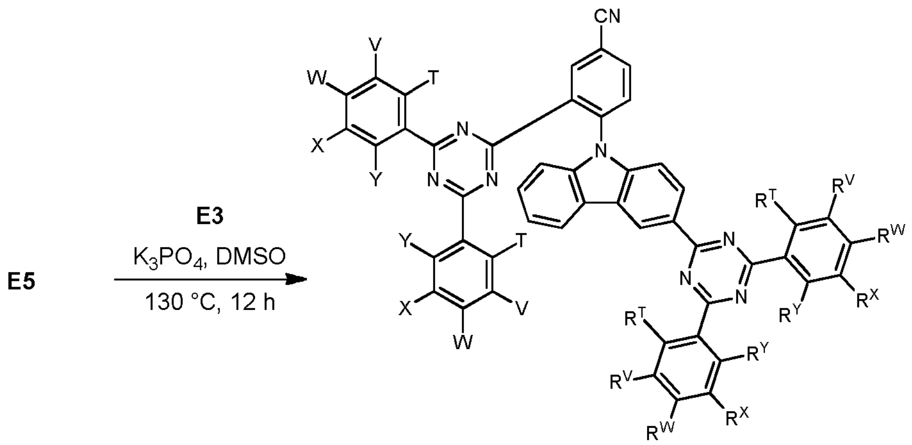


**E5** (1.00 equivalent), **E3** (1.00 equivalent) and tribasic potassium phosphate (2.0 equivalents) are suspended under nitrogen atmosphere in DMSO and stirred at 140 °C for 12 h. After cooling down to room temperature (RT), the reaction mixture is poured in water and extracted with ethyl acetate. The organic layer is dried over MgSO4, filtered and concentrated. The crude product is purified by flash chromatography. The product is obtained as solid.

*Cyclic voltammetry*

Cyclic voltammograms are measured from solutions having concentration of $10^{-3}$ mol/L of the organic molecules in dichloromethane or a suitable solvent and a suitable supporting electrolyte (e.g. 0.1 mol/L of tetrabutylammonium hexafluorophosphate). The measurements are conducted at room temperature under nitrogen atmosphere with a three-electrode assembly (Working and counter electrodes: Pt wire, reference electrode: Pt wire) and calibrated using $FeCp_2/FeCp_2^+$ as internal standard. The HOMO data was corrected using ferrocene as internal standard against a saturated calomel electrode (SCE).

*Density functional theory calculation*

Molecular structures are optimized employing the BP86 functional and the resolution of identity approach (RI). Excitation energies are calculated using the (BP86) optimized structures employing Time-Dependent DFT (TD-DFT) methods. Orbital and excited state energies are calculated with the B3LYP functional. Def2-SVP basis sets (and a m4-grid for numerical integration are used. The Turbomole program package is used for all calculations.

*Photophysical measurements*

Sample pretreatment: Spin-coating
Apparatus: Spin150, SPS euro.

The sample concentration is 1 mg/ml, dissolved in a suitable solvent. Program: 1) 3 s at 400 rpm; 20 s at 1000 rpm at 1000 rpm/s. 3) 10 s at 4000 rpm at 1000 rpm/s. After coating, the films are tried at 70 °C for 1 min.

Photoluminescence spectroscopy and TCSPC (*Time-correlated single-photon counting*) Steady-state emission spectroscopy is measured by a Horiba Scientific, Modell FluoroMax-4 equipped with a 150 W Xenon-Arc lamp, excitation- and emissions monochromators and a Hamamatsu R928 photomultiplier and a time-correlated single-photon counting option. Emissions and excitation spectra are corrected using standard correction fits.

Excited state lifetimes are determined employing the same system using the TCSPC method with FM-2013 equipment and a Horiba Y von TCSPC hub.

Excitation sources:

NanoLED 370 (wavelength: 371 nm, puls duration: 1,1 ns)
NanoLED 290 (wavelength: 294 nm, puls duration: <1 ns)
SpectraLED 310 (wavelength: 314 nm)
SpectraLED 355 (wavelength: 355 nm).

Data analysis (exponential fit) is done using the software suite DataStation and DAS6 analysis software. The fit is specified using the chi-squared-test.

Time-resolved PL spectrospcopy in the $\mu$s-range

Time-resolved PL measurements were performed on a FS5 fluorescence spectrometer from Edinburgh Instruments. Compared to measurements on the HORIBA setup, better light gathering allows for an optimized signal to noise ratio, which favors the FS5 system especially for transient PL measurements of delayed fluorescence characteristics. The FS5 consists of a xenon lamp providing a broad spectrum. The continuous light source is a 150W xenon arc lamp, selected wavelengths are chosen by a Czerny-Turner monochromator, which is also used to set specific emission wavelengths. The sample emission is directed towards a sensitive R928P photomultiplier tube (PMT), allowing the detection of single photons with a peak quantum efficiency of up to 25 % in the spectral range between 200 nm to 870 nm. The detector is a temperature stabilized PMT, providing dark counts below 300 cps (counts per second). Finally, to determine the transient decay lifetime of the delayed fluorescence, a tail fit using three exponential functions is applied. By weighting the specific lifetimes $\tau_i$ with their corresponding amplitudes $A_i$,

$$\tau_{\mathrm{DF}} = \sum_{i=1}^{3} \frac{A_i \tau_i}{A_i}$$

the delayed fluorescence lifetime $\tau_{\mathrm{DF}}$ is determined.

Photoluminescence quantum yield measurements

For photoluminescence quantum yield (PLQY) measurements an *Absolute PL Quantum Yield Measurement*

*C9920-03G* system (*Hamamatsu Photonics*) is used. Quantum yields and CIE coordinates are determined using the software U6039-05 version 3.6.0.

Emission maxima are given in nm, quantum yields Φ in % and CIE coordinates as x,y-values. PLQY is determined using the following protocol:

1) Quality assurance: Anthracene in ethanol (known concentration) is used as reference
2) Excitation wavelength: the absorption maximum of the organic molecule is determined, and the molecule is excited using this wavelength
3) Measurement

Quantum yields are measured for sample of solutions or films under nitrogen atmosphere. The yield is calculated using the equation:

$$\Phi_{PL} = \frac{n_{photon}, emited}{n_{photon}, absorbed} = \frac{\int \frac{\lambda}{hc}\left[Int_{emitted}^{sample}(\lambda) - Int_{absorbed}^{sample}(\lambda)\right]d\lambda}{\int \frac{\lambda}{hc}\left[Int_{emitted}^{reference}(\lambda) - Int_{absorbed}^{reference}(\lambda)\right]d\lambda}$$

wherein $n_{photon}$ denotes the photon count and Int. the intensity.

**Production and characterization of optoelectronic devices**

OLED devices comprising organic molecules according to the invention can be produced via vacuum-deposition methods. If a layer contains more than one compound, the weight-percentage of one or more compounds is given in %. The total weight-percentage values amount to 100 %, thus if a value is not given, the fraction of this compound equals to the difference between the given values and 100 %.

The not fully optimized OLEDs are characterized using standard methods and measuring electroluminescence spectra, the external quantum efficiency (in %) in dependency on the intensity, calculated using the light detected by the photodiode, and the current. The OLED device lifetime is extracted from the change of the luminance during operation at constant current density. The LT50 value corresponds to the time, where the measured luminance decreased to 50 % of the initial luminance, analogously LT80 corresponds to the time point, at which the measured luminance decreased to 80 % of the initial luminance, LT 95 to the time point, at which the measured luminance decreased to 95 % of the initial luminance etc.

Accelerated lifetime measurements are performed (e.g. applying increased current densities). Exemplarily LT80 values at 500 cd/m$^2$ are determined using the following equation:

$$\mathrm{LT80}\left(500\frac{cd^2}{m^2}\right) = \mathrm{LT80}(L_0)\left(\frac{L_0}{500\frac{cd^2}{m^2}}\right)^{1.6}$$

wherein $L_0$ denotes the initial luminance at the applied current density.

The values correspond to the average of several pixels (typically two to eight), the standard deviation between these pixels is given.

**HPLC-MS**

HPLC-MS analysis is performed on an HPLC by Agilent (1100 series) with MS-detector (Thermo LTQ XL).

Exemplary a typical HPLC method is as follows: a reverse phase column 4,6mm x 150mm, particle size 3,5 μm from Agilent *(ZORBAX Eclipse Plus 95Å C18, 4.6 × 150 mm, 3.5 μm HPLC column)* is used in the HPLC. The HPLC-MS measurements are performed at room temperature (rt) following gradients

| Flow rate [ml/min] | time [min] | A[%] | B[%] | C[%] |
|---|---|---|---|---|
| 2.5 | 0 | 40 | 50 | 10 |
| 2.5 | 5 | 40 | 50 | 10 |

(continued)

| Flow rate [ml/min] | time [min] | A[%] | B[%] | C[%] |
|---|---|---|---|---|
| 2.5 | 25 | 10 | 20 | 70 |
| 2.5 | 35 | 10 | 20 | 70 |
| 2.5 | 35.01 | 40 | 50 | 10 |
| 2.5 | 40.01 | 40 | 50 | 10 |
| 2.5 | 41.01 | 40 | 50 | 10 |

using the following solvent mixtures:

| | | |
|---|---|---|
| solvent A: | $H_2O$ (90%) | MeCN (10%) |
| solvent B: | $H_2O$ (10%) | MeCN (90%) |
| solvent C: | THF (50%) | MeCN (50%) |

An injection volume of 5 µL from a solution with a concentration of 0.5 mg/mL of the analyte is taken for the measurements.

Ionization of the probe is performed using an APCI (atmospheric pressure chemical ionization) source either in positive (APCI +) or negative (APCI -) ionization mode.

**Example 1**

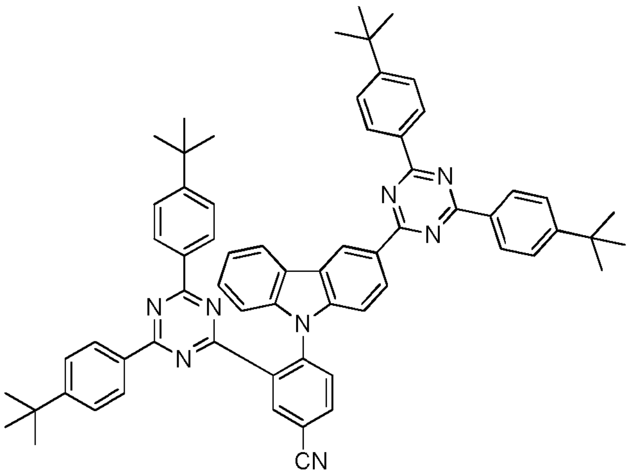

Example **1** was synthesized according to

***AAV1*** (yield 65 %), wherein cyanuric chloride (CAS 108-77-0) and 1-bromo-4-*tert*-butylbenzene (CAS 3972-65-4) were used as reactants,

***AAV2*** (yield 56 %), 3-(4,4,5,5-tetramethyl-1,3,2-dioxaborolan-2-yl)carbazole (CAS 855738-89-5) and 2-chloro-4,6-di-(4-*tert*-butylphenyl)triazine were used as reactans,

***AAV3*** (yield 79 %), wherein 3-bromo-4-fluorobenzonitrile (CAS 79630-23-2) and bis-(pinacolato)diboron (CAS 73183-34-3) were used as reactans,

***AAV4*** (yield 38 %), wherein 3-(4,4,5,5-tetramethyl-1,3,2-dioxaborolan-2-yl)-4-fluoro benzonitrile and 2-chloro-4,6-di-(4-*tert*-butylphenyl)triazine

***AAV5*** (yield 31 %), wherein 3-[4,6-di-(4-*tert*-butylphenyl)triazinyl]carbazole and 3-[4,6-di(4-*tert*-butylphenyl]triazinyl)-4-fluoro-benzonitrile were used as reactans.

HPLC-LCMS:

| Molecular Formula | m/z calculated | m/z found |
|---|---|---|
| $C_{65}H_{62}N_8$ | 955.26 | 954.66 |

Figure 1 depicts the emission spectrum of example **1** (10 % by weight in PMMA). The emission maximum ($\lambda_{max}$) is at 462 nm. The photoluminescence quantum yield (PLQY) is 63 %, the full width at half maximum (FWHM) is 0.42 eV. The resulting $CIE_x$ coordinate is determined at 0.16 and the $CIE_y$ coordinate at 0.18. The HOMO(E) is -6.14 eV.

**Example 2**

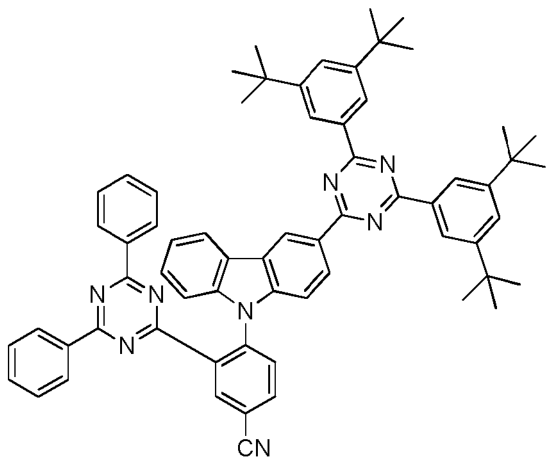

Example **2** was synthesized according to

***AAV1*** (yield 52 %), wherein cyanuric chloride (CAS 108-77-0) and 1-bromo-3,5-di-*tert*-butylbenzene (CAS 22385-77-9) were used as reactants,
***AAV2*** (yield 56 %), 3-(4,4,5,5-tetramethyl-1,3,2-dioxaborolan-2-yl)carbazole (CAS 855738-89-5) and 2-chloro-4,6-di-(4-*tert*-butylphenyl)triazine were used as reactans,
***AAV3*** (yield 79 %), wherein 3-bromo-4-fluorobenzonitrile (CAS 79630-23-2) and bis-(pinacolato)diboron (CAS 73183-34-3) were used as reactans,
***AAV4*** (yield 67 %), wherein 3-(4,4,5,5-tetramethyl-1,3,2-dioxaborolan-2-yl)-4-fluoro benzonitrile and 2-chloro-4,6-diphenyl-1,3,5-triazine (CAS 3842-55-5)
***AAV5*** (yield 26 %), wherein 3-[4,6-di-(3,5-di-*tert*-phenyl)triazinyl]carbazole and 3-(4,6-diphenyl triazinyl)-4-fluoro-benzonitrile were used as reactans.

HPLC-LCMS:

| Molecular Formula | m/z calculated | m/z found |
|---|---|---|
| $C_{65}H_{62}N_8$ | 955.26 | 954.66 |

Figure 2 depicts the emission spectrum of example **2** (10 % by weight in PMMA). The emission maximum ($\lambda_{max}$) is at 465 nm. The photoluminescence quantum yield (PLQY) is 67 %, the full width at half maximum (FWHM) is 0.41 eV. The resulting $CIE_x$ coordinate is determined at 0.16 and the $CIE_y$ coordinate at 0.21 and HOMO(E) is -6.19 eV.

**Example 3**

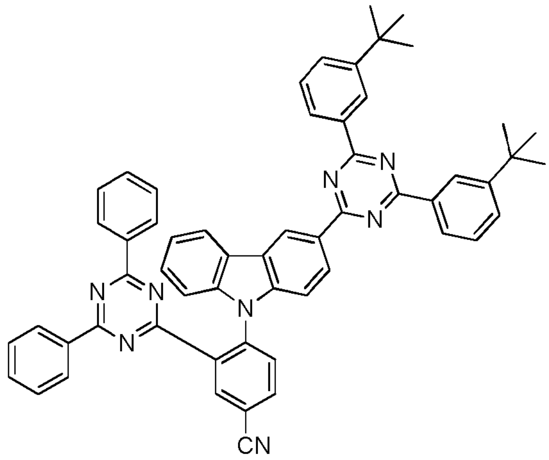

Example **3** was synthesized according to

***AAV1*** (yield 37 %), wherein cyanuric chloride (CAS 108-77-0) and 1-bromo-3-*tert*-butylbenzene (CAS 3972-64-3) were used as reactants,
***AAV2*** (yield 23 %), 3-(4,4,5,5-tetramethyl-1,3,2-dioxaborolan-2-yl)carbazole (CAS:855738-89-5) and 2-chloro-4,6-di-(3-*tert*-butylphenyl)triazine were used as reactans,
***AAV3*** (yield 79 %), wherein 3-bromo-4-fluorobenzonitrile (CAS 79630-23-2) and bis-(pinacolato)diboron (CAS 73183-34-3) were used as reactans,
***AAV4*** (yield 67 %), wherein 3-(4,4,5,5-tetramethyl-1,3,2-dioxaborolan-2-yl)-4-fluoro benzonitrile and 2-chloro-4,6-diphenyl-1,3,5-triazine (CAS 3842-55-5)
***AAV5*** (yield 44 %), wherein 3-[4,6-di-(3-*tert*-phenyl)triazinyl]carbazole and 3-(4,6-diphenyl triazinyl)-4-fluoro-benzonitrile were used as reactans.

HPLC-LCMS:

| Molecular Formula | m/z calculated | m/z found |
|---|---|---|
| $C_{57}H_{46}N_8$ | 843.02 | 842.53 |

Figure 3 depicts the emission spectrum of example **3** (10 % by weight in PMMA). The emission maximum ($\lambda_{max}$) is at 465 nm. The photoluminescence quantum yield (PLQY) is 68 %, the full width at half maximum (FWHM) is 0.41 eV. The resulting $CIE_x$ coordinate is determined at 0.16 and the $CIE_y$ coordinate at 0.21. The energy level of the highest occupied molecular orbital HOMO(E) is -6.16 eV.

**Example 4**

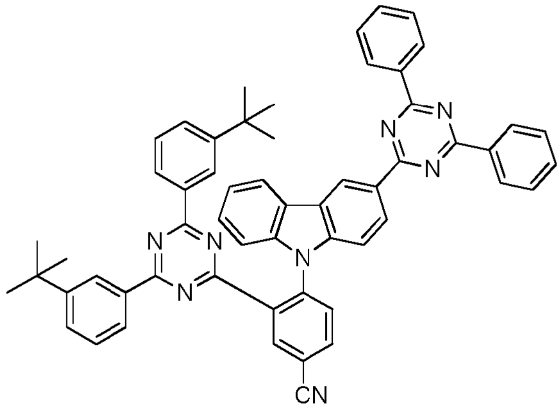

Example **4** was synthesized according to

***AAV1*** (yield 37 %), wherein cyanuric chloride (CAS 108-77-0) and 1-bromo-3-*tert*-butylbenzene (CAS 3972-64-3) were used as reactants,
***AAV2*** (yield 40 %), 3-(4,4,5,5-tetramethyl-1,3,2-dioxaborolan-2-yl)carbazole (CAS 855738-89-5) and 2-chloro-4,6-diphenyl-1,3,5-triazine (CAS 3842-55-5) were used as reactans,
***AAV3*** (yield 79 %), wherein 3-bromo-4-fluorobenzonitrile (CAS 79630-23-2) and bis-(pinacolato)diboron (CAS 73183-34-3) were used as reactans,
***AAV4*** (yield 82 %), wherein 3-(4,4,5,5-tetramethyl-1,3,2-dioxaborolan-2-yl)-4-fluoro benzonitrile and 2-chloro-4,6-di-(3-*tert*-butylphenyl)triazine
***AAV5*** (yield 7 %), wherein 3-(4,6-diphenyltriazinyl)carbazole and 3-(4,6-di-(3-*tert*-butylphenyl) triazinyl)-4-fluorobenzonitrile were used as reactans.

HPLC-LCMS:

| Molecular Formula | m/z calculated | m/z found |
|---|---|---|
| $C_{57}H_{46}N_8$ | 843.05 | 842.34 |

Figure 4 depicts the emission spectrum of example **4** (10 % by weight in PMMA). The emission maximum ($\lambda_{max}$) is at 460 nm. The photoluminescence quantum yield (PLQY) is 56 %, the full width at half maximum (FWHM) is 0.42 eV. The resulting $CIE_x$ coordinate is determined at 0.16 and the $CIE_y$ coordinate at 0.18.

**Example 5**

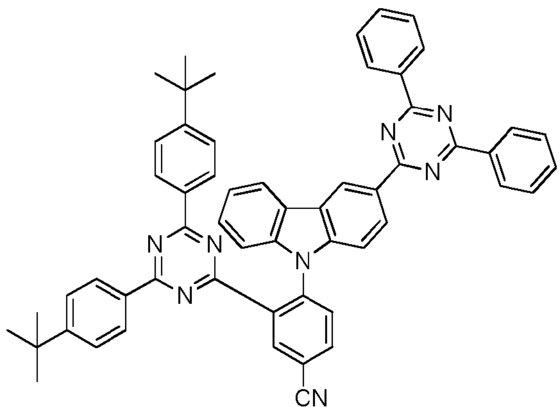

Example **5** was synthesized according to

***AAV1*** (yield 65 %), wherein cyanuric chloride (CAS 108-77-0) and 1-bromo-4-*tert*-butylbenzene (CAS 3972-65-4) were used as reactants,
***AAV2*** (yield 40 %), 3-(4,4,5,5-tetramethyl-1,3,2-dioxaborolan-2-yl)carbazole (CAS 855738-89-5) and 2-chloro-4,6-diphenyl-1,3,5-triazine (CAS 3842-55-5) were used as reactans,
***AAV3*** (yield 79 %), wherein 3-bromo-4-fluorobenzonitrile (CAS 79630-23-2) and bis-(pinacolato)diboron (CAS 73183-34-3) were used as reactans,
***AAV4*** (yield 38 %), wherein 3-(4,4,5,5-tetramethyl-1,3,2-dioxaborolan-2-yl)-4-fluoro benzonitrile and 2-chloro-4,6-di(4-*tert*-butylphenyl)triazine,
***AAV5*** (yield 43 %), wherein 3-(4,6-diphenyltriazinyl)carbazole and 3-(4,6-di(4-*tert*-butylphenyl) triazinyl)-4-fluoro-benzonitrile were used as reactants.

HPLC-LCMS:

| Molecular Formula | m/z calculated | m/z found |
|---|---|---|
| $C_{57}H_{46}N_8$ | 843.05 | 842.42 |

The energy level of the highest occupied molecular orbital HOMO(E) of example **5** is -6.16 eV.

**Example 6**

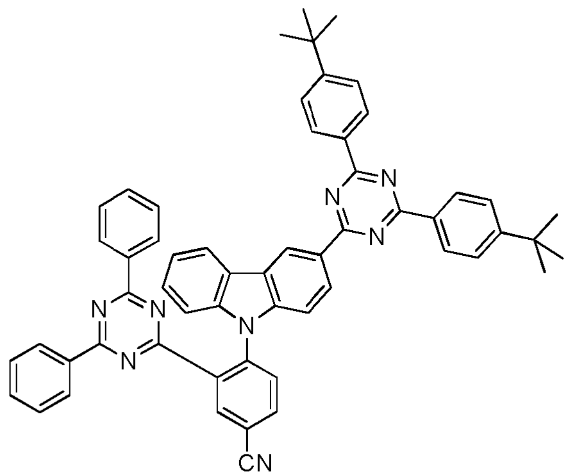

Example **6** was synthesized according to

***AAV1*** (yield 65 %), wherein cyanuric chloride (CAS 108-77-0) and 1-bromo-4-*tert*-butylbenzene (CAS 3972-65-4) were used as reactants,
***AAV2*** (yield 56 %), 3-(4,4,5,5-tetramethyl-1,3,2-dioxaborolan-2-yl)carbazole (CAS 855738-89-5) and 2-chloro-4,6-di(4-*tert*-butylphenyl)triazine were used as reactans,
***AAV3*** (yield 79 %), wherein 3-bromo-4-fluorobenzonitrile (CAS 79630-23-2) and bis-(pinacolato)diboron (CAS 73183-34-3) were used as reactans,
***AAV4*** (yield 67 %), wherein 3-(4,4,5,5-tetramethyl-1,3,2-dioxaborolan-2-yl)-4-fluoro benzonitrile and 2-chloro-4,6-diphenyl-1,3,5-triazine (CAS 3842-55-5)
***AAV5*** (yield 10 %), wherein 3-[4,6-di(4-*tert*-phenyl)triazinyl]carbazole and 3-(4,6-diphenyl triazinyl)-4-fluoro-benzonitrile were used as reactans.

HPLC-LCMS:

| Molecular Formula | m/z calculated | m/z found |
|---|---|---|
| $C_{57}H_{46}N_8$ | 843.05 | 842.55 |

Figure 5 depicts the emission spectrum of example **6** (10 % by weight in PMMA). The emission maximum ($\lambda_{max}$) is at 466 nm. The photoluminescence quantum yield (PLQY) is 68 %, the full width at half maximum (FWHM) is 0.42 eV. The resulting $CIE_x$ coordinate is determined at 0.16 and the $CIE_y$ coordinate at 0.21.

**Example D1**

Example **2** was tested in the OLED **D1**, which was fabricated with the following layer structure:

| Layer | Thickness | D1 |
|---|---|---|
| **10** | 100 nm | Al |
| **9** | 2 nm | Liq |
| **8** | 20 nm | NBPhen |
| **7** | 10 nm | MAT1 |
| **6** | 50 nm | Example **2** (10%): mCBP (70%) : MAT1 (20%) |
| **5** | 10 nm | mCBP |
| **4** | 10 nm | TCTA |
| **3** | 40 nm | NPB |
| **2** | 5 nm | HAT-CN |
| **1** | 50 nm | ITO |
| **Substrate** | | glass |

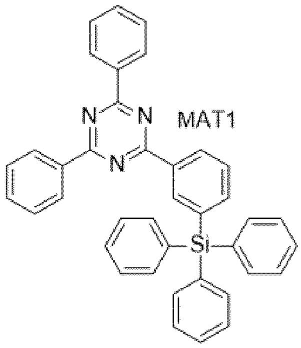
MAT1

Device **D1** yielded an external quantum efficiency (EQE) at 1000 cd/m$^2$ of 15.8 $\pm$ 0.2%. The emission maximum is at 468 nm with a FWHM of 60 nm at 5.9 V. The corresponding CIEx value is 0.14 and CIEy is 0.19.

**Example D2**

Example 3 was tested in the OLED **D2**, which was fabricated with the following layer structure:

| Layer | Thickness | D2 |
|---|---|---|
| **10** | 100 nm | Al |
| **9** | 2 nm | Liq |
| **8** | 20 nm | NBPhen |

(continued)

| Layer | Thickness | D2 |
|---|---|---|
| **7** | 10 nm | MAT1 |
| **6** | 50 nm | Example **3** (10%): mCBP (70%) : MAT1 (20%) |
| **5** | 10 nm | mCBP |
| **4** | 10 nm | TCTA |
| **3** | 40 nm | NPB |
| **2** | 5 nm | HAT-CN |
| **1** | 50 nm | ITO |
| **Substrate** | | glass |

Device **D2** yielded an external quantum efficiency (EQE) at 1000 cd/m$^2$ of 16.6 $\pm$ 0.2%. The emission maximum is at 468 nm with a FWHM of 63 nm at 5.4 V. The corresponding CIEx value is 0.15 and CIEy is 0.21.

**Example D3**

Example **4** was tested in the OLED **D3**, which was fabricated with the following layer structure:

| Layer | Thickness | D3 |
|---|---|---|
| **10** | 100 nm | Al |
| **9** | 2 nm | Liq |
| **8** | 20 nm | NBPhen |
| **7** | 10 nm | MAT1 |
| **6** | 50 nm | Example **4** (10%): mCBP (70%) : MAT1 (20%) |
| **5** | 10 nm | mCBP |
| **4** | 10 nm | TCTA |
| **3** | 40 nm | NPB |
| **2** | 5 nm | HAT-CN |
| **1** | 50 nm | ITO |
| **Substrate** | | glass |

Device **D3** yielded an external quantum efficiency (EQE) at 1000 cd/m$^2$ of 15.6 $\pm$ 0.1%. The emission maximum is at 467 nm with a FWHM of 61 nm at 5.5V. The corresponding CIEx value is 0.15 and CIEy is 0.18.

**Example D4**

Example **5** was tested in the OLED **D4,** which was fabricated with the following layer structure:

| Layer | Thickness | D4 |
|---|---|---|
| **10** | 100 nm | Al |
| **9** | 2 nm | Liq |
| **8** | 20 nm | NBPhen |
| **7** | 10 nm | MAT1 |
| **6** | 50 nm | Example **5** (10%): mCBP (70%) : MAT1 (20%) |

(continued)

| Layer | Thickness | D4 |
|---|---|---|
| **5** | 10 nm | mCBP |
| **4** | 10 nm | TCTA |
| **3** | 40 nm | NPB |
| **2** | 5 nm | HAT-CN |
| **1** | 50 nm | ITO |
| **Substrate** | | glass |

Device **D4** yielded an external quantum efficiency (EQE) at 1000 cd/m$^2$ of 14.3 $\pm$ 0.3%. The emission maximum is at 468 nm with a FWHM of 63 nm at 4.7 V. The corresponding CIEx value is 0.15 and CIEy is 0.20.

**Example D5**

Example **6** was tested in the OLED **D5,** which was fabricated with the following layer structure:

| Layer | Thickness | D5 |
|---|---|---|
| **10** | 100 nm | Al |
| **9** | 2 nm | Liq |
| **8** | 20 nm | NBPhen |
| **7** | 10 nm | MAT1 |
| **6** | 50 nm | Example **6** (20%): mCBP (80%) |
| **5** | 10 nm | mCBP |
| **4** | 10 nm | TCTA |
| **3** | 40 nm | NPB |
| **2** | 5 nm | HAT-CN |
| **1** | 50 nm | ITO |
| **Substrate** | | Glass |

Device **D5** yielded an external quantum efficiency (EQE) at 1000 cd/m$^2$ of 15.6 $\pm$ 0.1%. The emission maximum is at 467 nm with a FWHM of 60 nm at 4.9 V. The corresponding CIEx value is 0.15 and CIEy is 0.21.

**Example D6**

Example **1** was tested in the OLED **D6,** which was fabricated with the following layer structure:

| Layer | Thickness | D5 |
|---|---|---|
| **10** | 100 nm | Al |
| **9** | 2 nm | Liq |
| **8** | 20 nm | NBPhen |
| **7** | 10 nm | MAT1 |
| **6** | 50 nm | Example **1** (15%): mCBP (84%) : MAT2 (1%) |
| **5** | 10 nm | mCBP |

MAT2

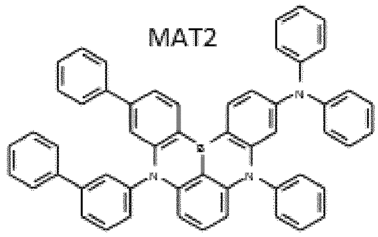

| | | |
|---|---|---|
| **4** | 10 nm | TCTA |
| **3** | 40 nm | NPB |
| **2** | 5 nm | HAT-CN |
| **1** | 50 nm | ITO |
| **Substrate** | | Glass |

The emission maximum of device **D6** is at 468 nm with a FWHM of 34 nm. The corresponding CIEx value is 0.15 and CIEy is 0.19.

**Comparative Example CD1**

Additionally, a comparative OLED **CD1** was fabricated with the layer structure of **D6,** wherein example **1** was replaced by MAT3:

| **Layer** | **Thickness** | **D5** | |
|---|---|---|---|
| **10** | 100 nm | Al | MAT3 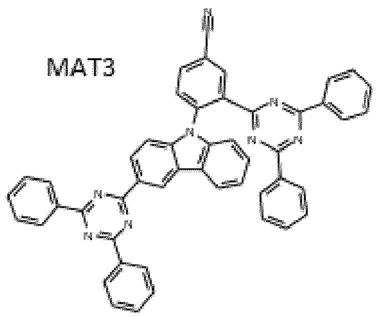 |
| **9** | 2 nm | Liq | |
| **8** | 20 nm | NBPhen | |
| **7** | 10 nm | MAT1 | |
| **6** | 50 nm | MAT3 (15%): mCBP (84%) : MAT2 (1%) | |
| **5** | 10 nm | mCBP | |
| **4** | 10 nm | TCTA | |
| **3** | 40 nm | NPB | |
| **2** | 5 nm | HAT-CN | |
| **1** | 50 nm | ITO | |
| **Substrate** | | Glass | |

The emission maximum of device **CD1** is at 468 nm with a FWHM of 40 nm. The corresponding CIEx value is 0.15 and CIEy is 0.24.

Comparing **D6** and **CD1,** it is observed that the color coordinates for the device containing the molecule of the invention are closer to the primary color blue, i.e. the CIEy coordinate is shifted to lower values while the CIEx coordinate remained the same when using an organic molecule of the invention. In general, it is expected that this finding holds true for all molecules according to Formula DAB-I used as the one or more emitter which differs from the organic molecule according to the invention.

**Figures**

The drawings of the figures show:

Figure 1 Emission spectrum of example **1** (10% by weight) in PMMA.
Figure 2 Emission spectrum of example **2** (10% by weight) in PMMA.
Figure 3 Emission spectrum of example **3** (10% by weight) in PMMA.
Figure 4 Emission spectrum of example **4** (10% by weight) in PMMA.
Figure 5 Emission spectrum of example **6** (10% by weight) in PMMA.

## Claims

1. Organic molecule, comprising a structure of formula I

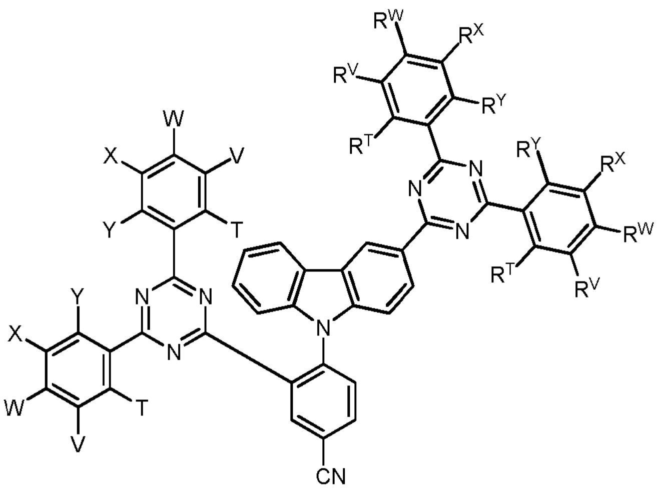

Formula I

wherein

T, V, W, X, Y is independently selected from the group consisting of H and tert-butyl;
$R^T$, $R^V$, $R^W$, $R^X$, $R^Y$ is independently selected from the group consisting of H and tert-butyl;
wherein at least one kind of substituent selected from the group consisting of T, V, W, X, Y, $R^T$, $R^V$, $R^W$, $R^X$, $R^Y$ is tert-butyl at each occurrence.

2. Organic molecule according to claim 1, wherein the organic molecule comprises a structure of formula Ia:

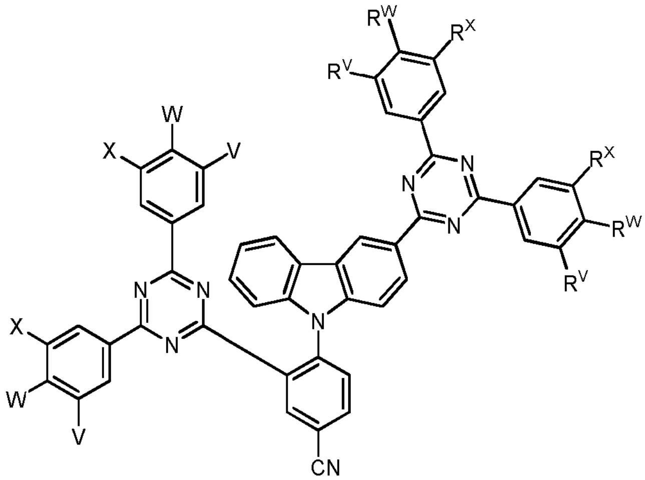

Formula Ia

wherein

at least one kind of substituents selected from the group consisting of V, W, X, $R^V$, $R^W$, $R^X$ is tert-butyl at each occurrence.

3. Organic molecule according to claim 1 or 2, wherein the organic molecule comprises a structure of formula Iaa:

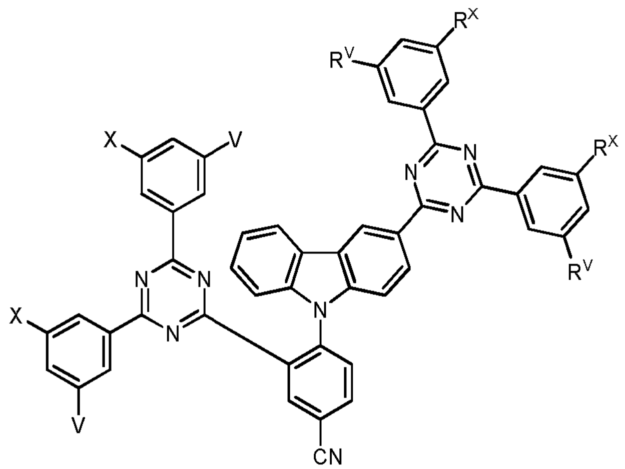

Formula Iaa

wherein
at least one kind of substituents selected from the group consisting of V, X, $R^V$, $R^X$ is tert-butyl at each occurrence.

4. Organic molecule according to one or more of claims 1 to 3, wherein the organic molecule comprises a structure of formula Iaaa:

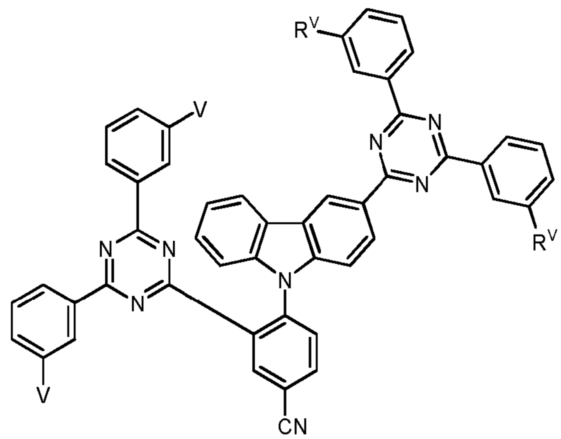

Formula Iaaa

wherein
at least one kind of substituents selected from the group consisting of V and $R^V$ is tert-butyl at each occurrence.

5. Use of an organic molecule according to one or more of claims 1 to 4 as a luminescent emitter and/or as a host material and/or as an electron transport material and/or as a hole injection material and/or as a hole blocking material in an optoelectronic device.

6. Use according to claim 5, wherein the optoelectronic device is selected from the group consisting of:

- organic light-emitting diodes (OLEDs),
- light-emitting electrochemical cells,
- OLED-sensors,

- organic diodes,
- organic solar cells,
- organic transistors,
- organic field-effect transistors,
- organic lasers, and
- down-conversion elements.

**7.** Composition, comprising:

(a) an organic molecule according to one or more of claims 1 to 4, in particular in the form of an emitter and/or a host, and
(b) an emitter and/or host material, which differs from the organic molecule, and
(c) optionally, a dye and/or a solvent.

**8.** Optoelectronic device, comprising an organic molecule according to one or more of claims 1 to 4 or a composition according to claim 7, in particular in form of a device selected from the group consisting of: organic light-emitting diode (OLED), light-emitting electrochemical cell, OLED-sensor, organic diode, organic solar cell, organic transistor, organic field-effect transistor, organic laser, and down-conversion element.

**9.** Optoelectronic device according to claim 8, comprising:

- a substrate,
- an anode, and
- a cathode, wherein the anode and/or the cathode are disposed on the substrate, and
- at least a light-emitting layer, which is arranged between the anode and the cathode and which comprises the organic molecule or the composition.

**10.** Method for producing an optoelectronic device, wherein an organic molecule according to any one of claims 1 to 4 or a composition according to claim 7 is used.

**11.** Method according to claim 10, comprising the processing of the organic molecule or of the composition by a vacuum evaporation method or from a solution.

## Patentansprüche

**1.** Organisches Molekül, aufweisend eine Struktur der Formel I

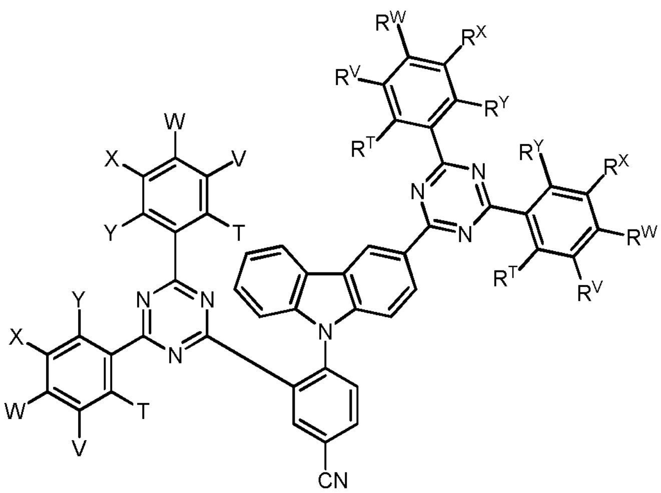

Formel I

wobei

T, V, W, X, Y unabhängig voneinander aus der Gruppe bestehend aus H und tert-Butyl ausgewählt sind; $R^T$, $R^V$, $R^W$, $R^X$, $R^Y$ unabhängig voneinander aus der Gruppe bestehend aus H und tert-Butyl ausgewählt sind; wobei mindestens eine Art von Substituent aus der Gruppe bestehend aus T, V, W, X, Y, $R^T$, $R^V$, $R^W$, $R^X$, $R^Y$ bei jedem Auftreten tert-Butyl ist.

**2.** Organisches Molekül nach Anspruch 1, wobei das organische Molekül eine Struktur der Formel Ia aufweist:

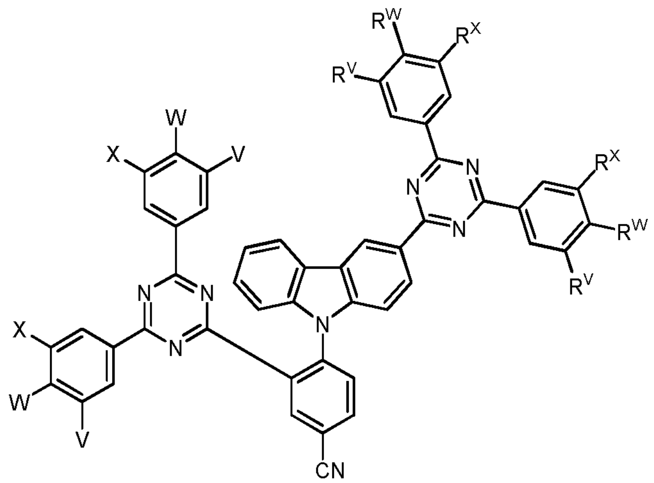

Formel Ia

wobei mindestens eine Art von Substituent aus der Gruppe bestehend aus V, W, X, $R^V$, $R^W$, $R^X$ bei jedem Auftreten tert-Butyl ist.

**3.** Organisches Molekül nach Anspruch 1 oder 2, wobei das organische Molekül eine Struktur der Formel Iaa aufweist:

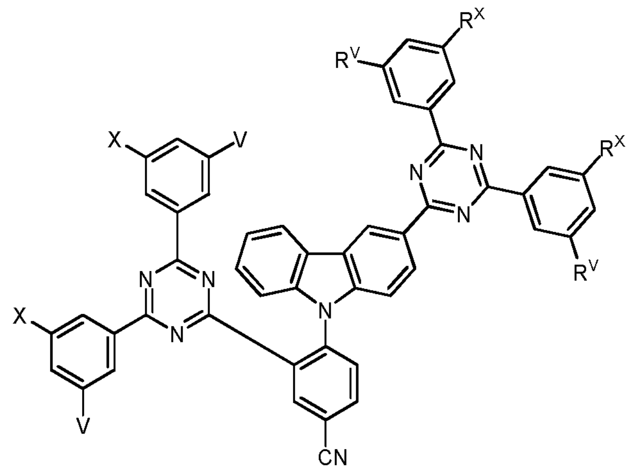

Formel Iaa

wobei

mindestens eine Art von Substituent aus der Gruppe bestehend aus V, X, $R^V$, $R^X$ bei jedem Auftreten tert-Butyl ist.

**4.** Organisches Molekül nach einem oder mehreren der Ansprüche 1 bis 3, wobei das organische Molekül eine Struktur der Formel Iaaa aufweist:

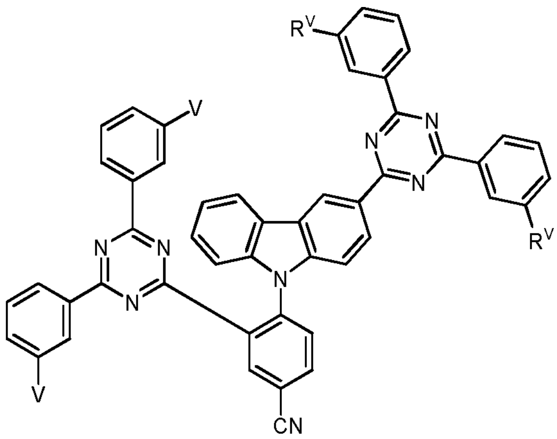

Formel Iaaa

wobei
mindestens eine Art von Substituent aus der Gruppe bestehend aus V und $R^V$ bei jedem Auftreten tert-Butyl ist.

**5.** Verwendung eines organischen Moleküls nach einem oder mehreren der Ansprüche 1 bis 4 als ein lumineszierender Emitter und/oder als ein Wirtsmaterial und/oder als ein Elektronentransportmaterial und/oder als ein Lochinjektionsmaterial und/oder als ein Lochblockiermaterial in einer optoelektronischen Vorrichtung.

**6.** Verwendung nach Anspruch 5, wobei die optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren,
- organischen Dioden,
- organischen Solarzellen,
- organischen Transistoren,
- organischen Feldeffekttransistoren,
- organischen Lasern und
- Down-Konversions-Elementen.

**7.** Zusammensetzung, aufweisend:

(a) ein organisches Molekül nach einem oder mehreren der Ansprüche 1 bis 4, insbesondere in Form eines Emitters und/oder eines Wirts, und
(b) ein Emitter- und/oder Wirtsmaterial, das von dem organischen Molekül verschieden ist, und
(c) optional ein Farbstoff und/oder ein Lösungsmittel.

**8.** Optoelektronische Vorrichtung, aufweisend ein organisches Molekül nach einem oder mehreren der Ansprüche 1 bis 4 oder eine Zusammensetzung nach Anspruch 7, insbesondere in Form einer Vorrichtung ausgewählt aus der Gruppe bestehend aus einer organischen lichtemittierenden Diode (OLED), einer lichtemittierenden elektrochemischen Zelle, einem OLED-Sensor, einer organischen Diode, einer organischen Solarzelle, einem organischen Transistor, einem organischen Feldeffekttransistor, einem organischen Laser und einem Down-Konversions-Element.

**9.** Optoelektronische Vorrichtung nach Anspruch 8, aufweisend:

- ein Substrat,
- eine Anode und
- eine Kathode, wobei die Anode und/oder die Kathode auf das Substrat aufgebracht ist, und
- mindestens eine lichtemittierende Schicht, die zwischen der Anode und der Kathode angeordnet ist und die das organische Molekül oder die Zusammensetzung aufweist.

10. Verfahren zur Herstellung einer optoelektronischen Vorrichtung, bei dem ein organisches Molekül nach einem der Ansprüche 1 bis 4 oder eine Zusammensetzung nach Anspruch 7 verwendet wird.

11. Verfahren nach Anspruch 10, aufweisend das Verarbeiten des organischen Moleküls oder der Zusammensetzung durch ein Vakuumverdampfungsverfahren oder aus einer Lösung.

**Revendications**

1. Molécule organique, comprenant une structure de formule I :

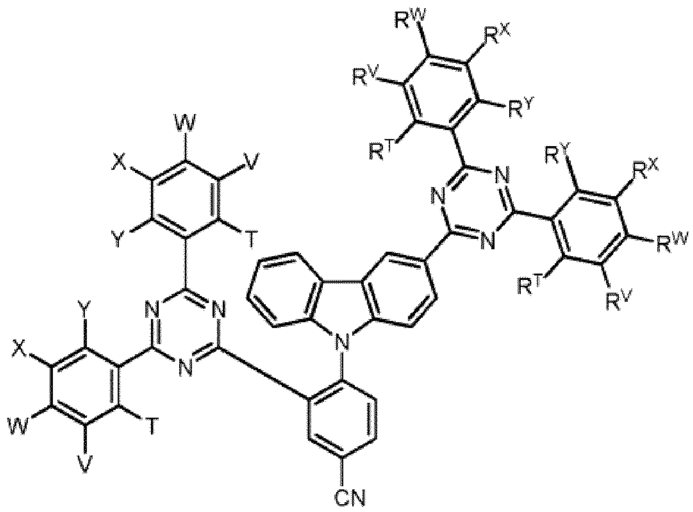

Formule I

dans laquelle

T, V, W, X, Y sont indépendamment choisis dans le groupe constitué par H et un tert-butyle ;
$R^T$, $R^V$, $R^W$, $R^X$, $R^Y$ sont indépendamment choisis dans le groupe constitué par H et un tert-butyle ;
dans laquelle au moins un type de substituant choisi dans le groupe constitué par T, V, W, X, Y, $R^T$, $R^V$, $R^W$, $R^X$, $R^Y$ est un tert-butyle à chaque occurrence.

2. Molécule organique selon la revendication 1, la molécule organique comprenant une structure de formule Ia :

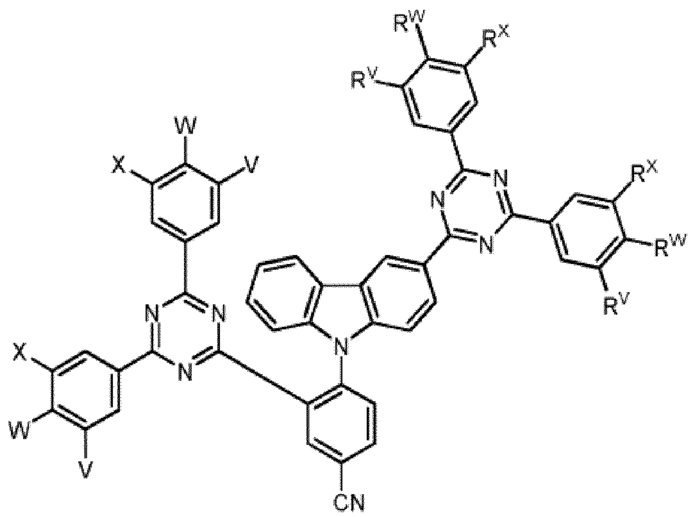

Formule Ia

dans laquelle au moins un type de substituant choisi dans le groupe constitué par V, W, X, $R^V$, $R^W$, $R^X$ est un tert-butyle à chaque occurrence.

**3.** Molécule organique selon la revendication 1 ou 2, la molécule organique comprenant une structure de formule Iaa :

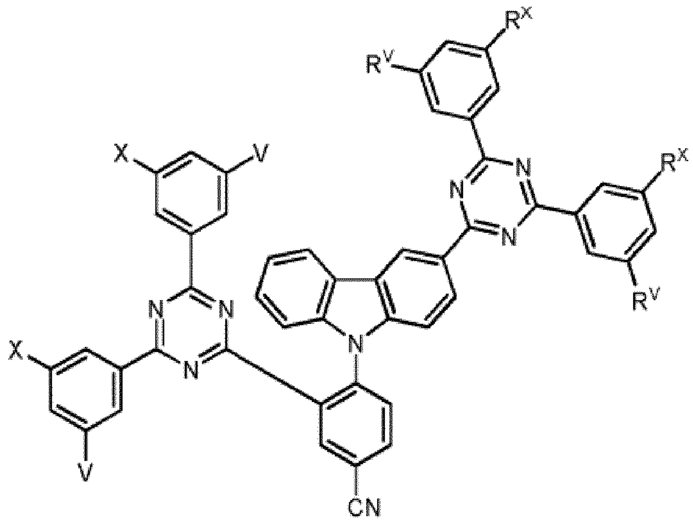

Formule Iaa

dans laquelle
au moins un type de substituant choisi dans le groupe constitué par V, X, $R^V$, $R^X$ est un tert-butyle à chaque occurrence.

**4.** Molécule organique selon une ou plusieurs des revendications 1 à 3, la molécule organique comprenant une structure de formule Iaaa :

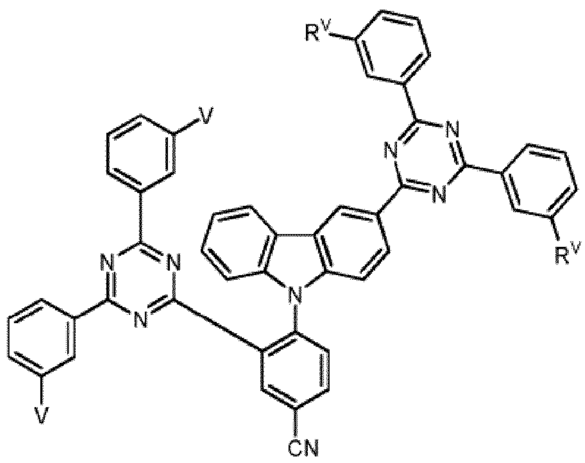

Formule Iaaa

dans laquelle
au moins un type de substituant choisi dans le groupe constitué par V et $R^V$ est un tert-butyle à chaque occurrence.

**5.** Utilisation d'une molécule organique selon une ou plusieurs des revendications 1 à 4 comme un émetteur luminescent et/ou comme un matériau hôte et/ou comme un matériau de transport d'électrons et/ou comme un matériau d'injection de trous et/ou comme un matériau de blocage de trous dans un dispositif optoélectronique.

**6.** Utilisation selon la revendication 5, dans laquelle le dispositif optoélectronique est choisi dans le groupe constitué par :

• les diodes électroluminescentes organiques (OLED),
• les cellules électrochimiques électroluminescentes,
• les capteurs OLED,
• les diodes organiques,
• les cellules solaires organiques,
• les transistors organiques,
• les transistors à effet de champ organiques,
• les lasers organiques, et
• les éléments de conversion descendante.

**7.** Composition, comprenant :

(a) une molécule organique selon une ou plusieurs des revendications 1 à 4, en particulier sous la forme d'un émetteur et/ou d'un hôte, et
(b) un matériau émetteur et/ou hôte, qui diffère de la molécule organique, et
(c) éventuellement, un colorant et/ou un solvant.

**8.** Dispositif optoélectronique, comprenant une molécule organique selon une ou plusieurs des revendications 1 à 4 ou une composition selon la revendication 7, en particulier sous la forme d'un dispositif choisi dans le groupe constitué par : une diode électroluminescente organique (OLED), une cellule électrochimique électroluminescente, un capteur OLED, une diode organique, une cellule solaire organique, un transistor organique, un transistor à effet de champ organique, un laser organique, et un élément de conversion descendante.

**9.** Dispositif optoélectronique selon la revendication 8, comprenant :

- un substrat,
- une anode, et
- une cathode, l'anode et/ou la cathode étant disposées sur le substrat, et
- au moins une couche électroluminescente, qui est disposée entre l'anode et la cathode et qui comprend la molécule organique ou la composition.

10. Procédé de production d'un dispositif optoélectronique, dans lequel une molécule organique selon l'une quelconque des revendications 1 à 4 ou une composition selon la revendication 7 est utilisée.

11. Procédé selon la revendication 10, comprenant le traitement de la molécule organique ou de la composition par un procédé d'évaporation sous vide ou à partir d'une solution.

Figure 1

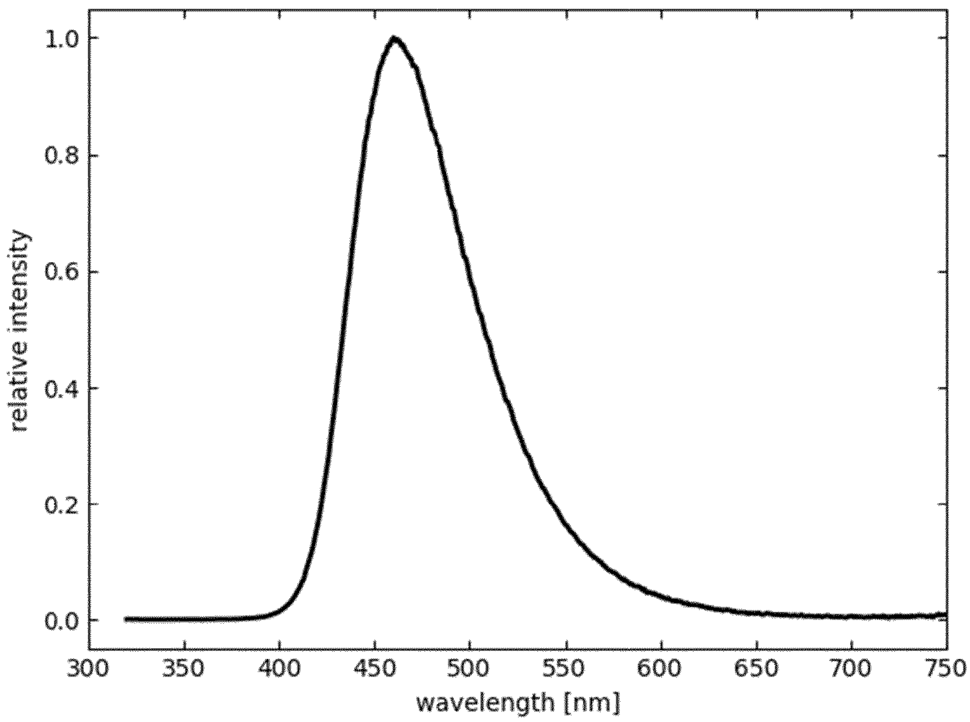


Figure 2

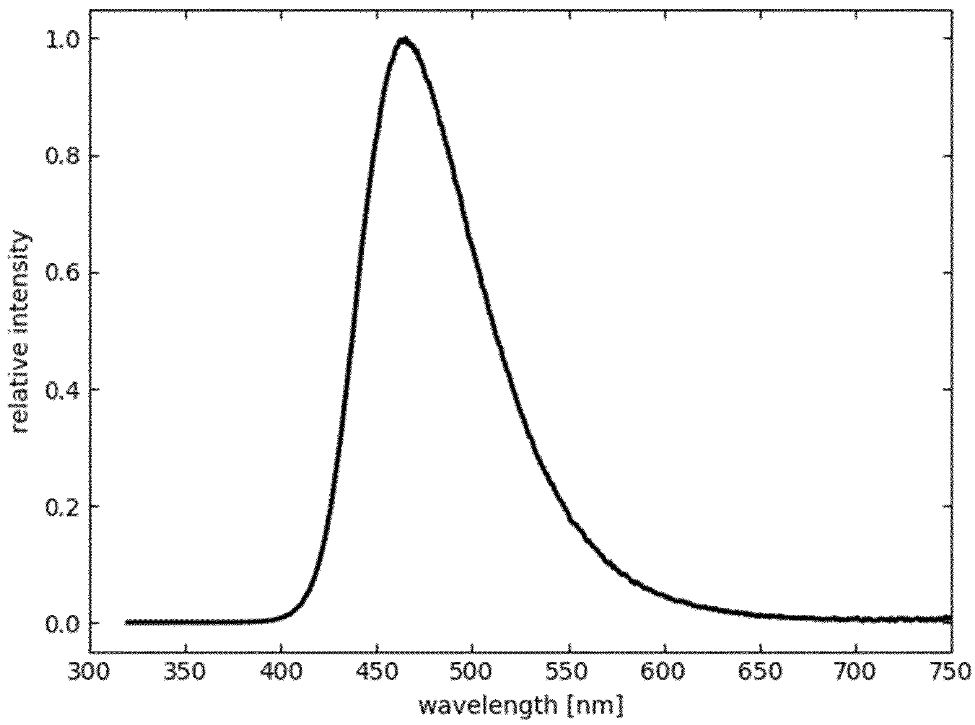

Figure 3

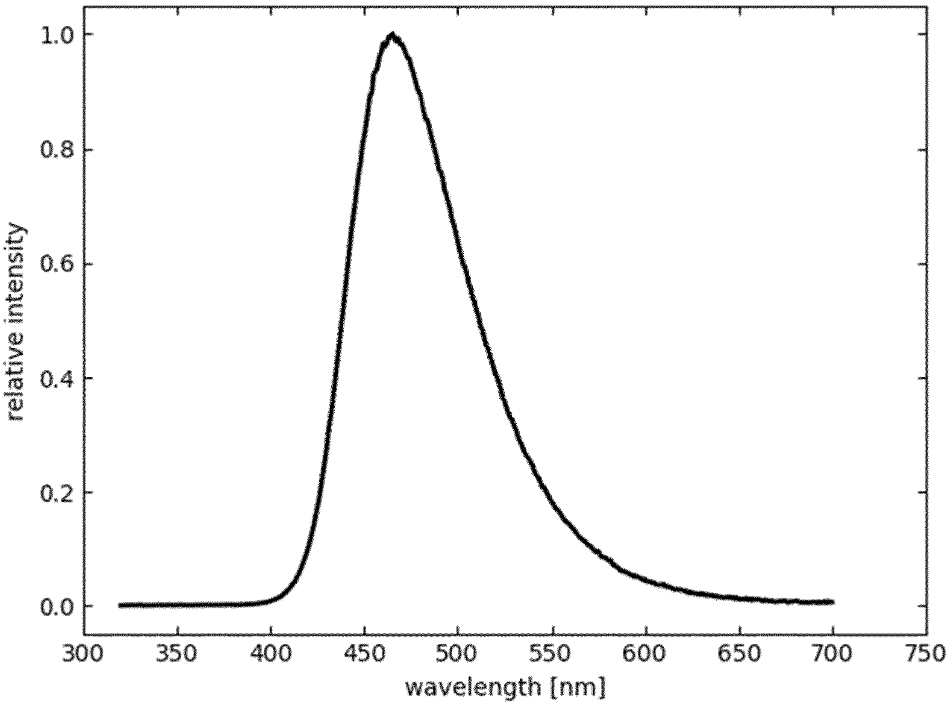


Figure 4

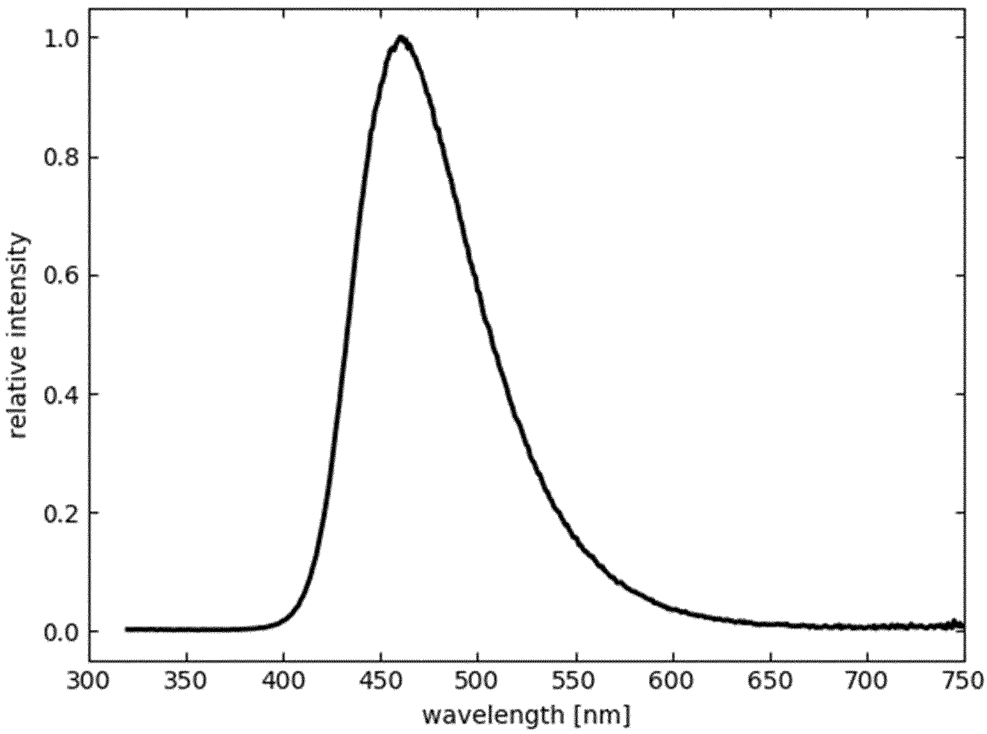

**Figure 5**

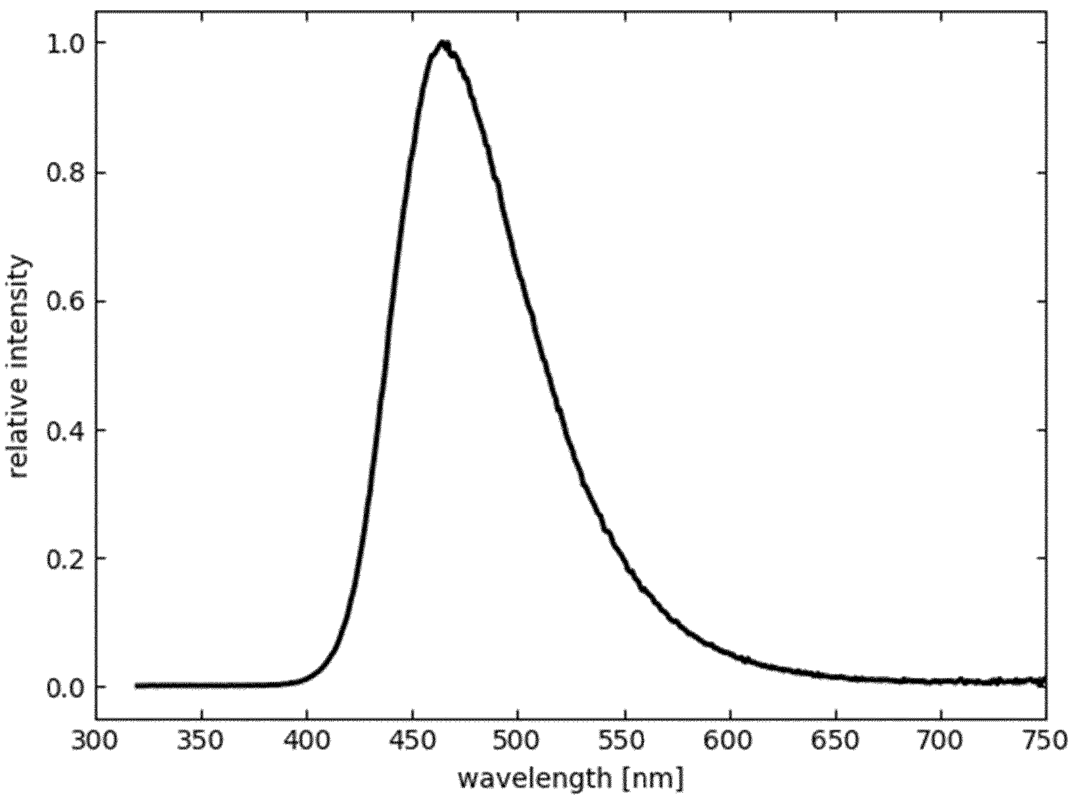
1.0
0.8
0.6
0.4
0.2
0.0
relative intensity
300
350
400
450
500
550
600
650
700
750
wavelength [nm]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3315581 A1
- US 2015236274 A


### Non-patent literature cited in the description


- *CHEMICAL ABSTRACTS,* 855738-89-5
- *CHEMICAL ABSTRACTS,* 14221-01-3
- *CHEMICAL ABSTRACTS,* 79630-23-2
- *CHEMICAL ABSTRACTS,* 73183-34-3
- *CHEMICAL ABSTRACTS,* 108-77-0
- *CHEMICAL ABSTRACTS,* 3972-65-4
- *CHEMICAL ABSTRACTS,* 22385-77-9
- *CHEMICAL ABSTRACTS,* 3842-55-5
- *CHEMICAL ABSTRACTS,* 3972-64-3